(19) Europäisches Patentamt
European Patent Office
Office européen des brevets

(11) **EP 1 742 161 A1**

(12) **EUROPEAN PATENT APPLICATION**

(43) Date of publication:
**10.01.2007 Bulletin 2007/02**

(51) Int Cl.:
***G06F 19/00*** *(2006.01)*

(21) Application number: **05256350.9**

(22) Date of filing: **13.10.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **04.07.2005 JP 2005195541**

(71) Applicant: **FUJITSU LIMITED**
**Kawasaki-shi,**
**Kanagawa 211-8588 (JP)**

(72) Inventors:
• **Yokoyama, Masaru**
**Sawara-ku**
**Fukuoka-shi**
**Fukuoka 814-8589 (JP)**
• **Kawahara, Yoshihiro**
**Sawara-ku**
**Fukuoka-shi**
**Fukuoka 814-8589 (JP)**

(74) Representative: **Hitching, Peter Matthew et al**
**HASELTINE LAKE**
**Imperial House**
**15-19 Kingsway**
**London WC2B 6UD (GB)**

(54) **Method, system and computer program for expression data analysis**

(57) A method for analysis in which a user can readily compare expression data and a pathway with a computer is provided as an alternative to subjective comparison or manual comparison of the expression data and the pathway according to certain criteria. Expression data of a protein/gene other than a target protein/gene is con- structed so as to fit in with a pathway on the basis of the target protein/gene expression data; and the protein/ gene expression data, in the constructed structure of the expression data, fitting in with the pathway is highlighted while the constructed structure of the expression data is displayed on a display.

FIG. 4

EP 1 742 161 A1

**Description**

The present invention relates to methods for comparing protein or gene expression data and pathways.

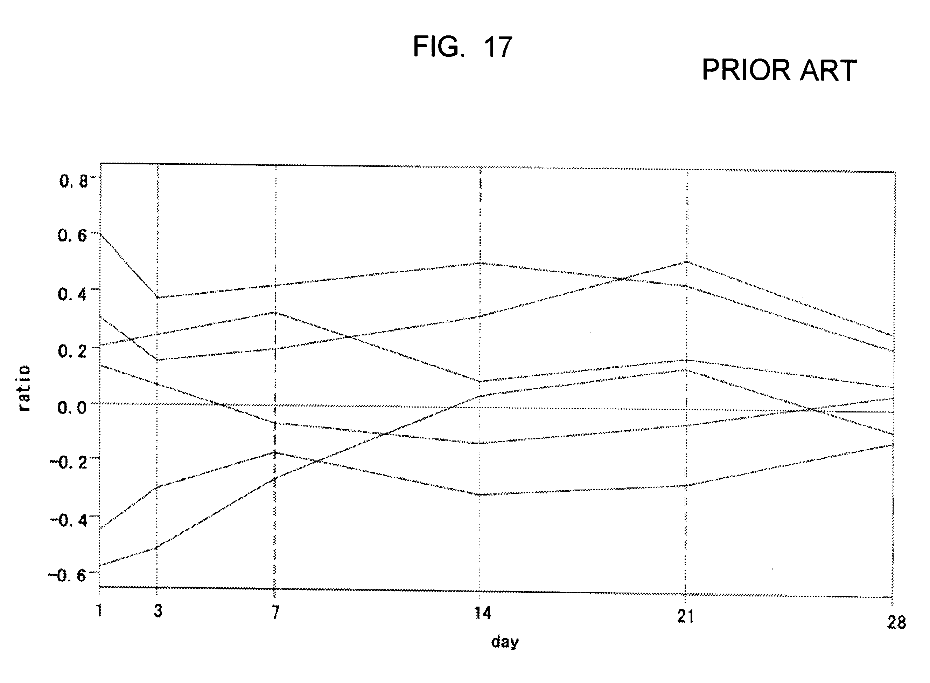

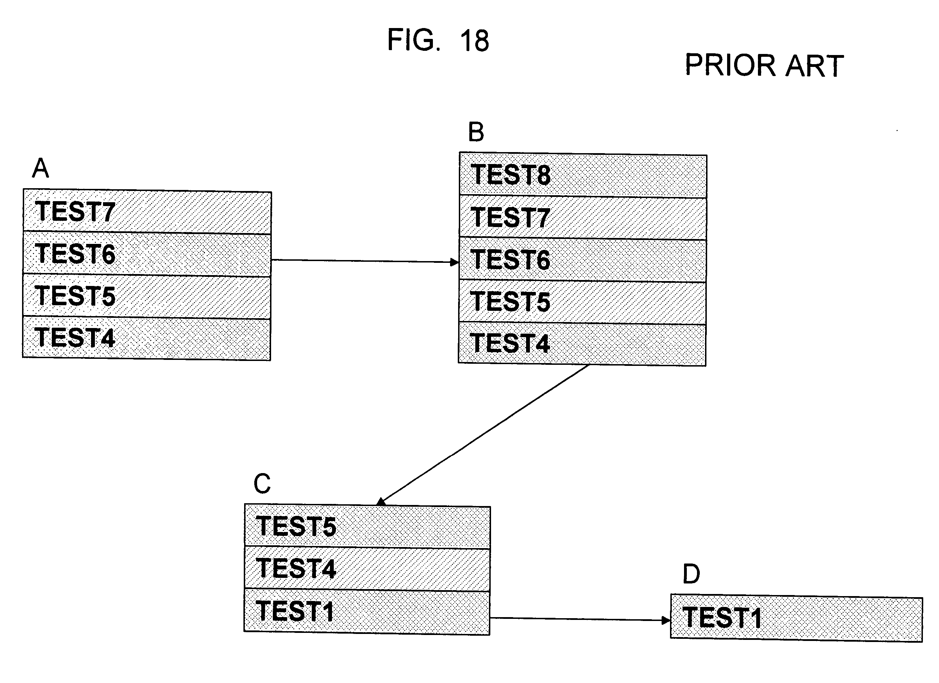

Time-series experimental data such as a gene expression profile is fixedly displayed as time-course graphs (time-varying axial line graph) or images (color-coded map of expression ratio). FIG. 17 is a time-course graph fixedly showing time-series data such as gene expression profiles. One line of the time-course graph indicates an expression ratio of one gene. FIG. 18 is a pathway diagram showing expression data of each gene at nodes of a pathway by color coding.

International Publication WO2002/025489 discloses a method for displaying gene data, which is another related art. This method for displaying gene data includes a step of displaying a plurality of gene expression patterns and a dendrogram obtained by cluster analysis of those expression patterns in such a manner as to correspond to each other; a step of specifying a function of a target gene and a distance on the dendrogram; and a step of highlighting a tree fragment of the dendrogram, wherein the tree fragment includes a gene having the specified function and is a route of a node having a distance not exceeding the specified distance from the gene on the dendrogram. According to this method for displaying gene data as a related art, expression pattern data of a plurality of genes can be displayed in a visually understandable manner and also in a manner that the functions and roles of the genes can be readily predicted. This is achieved by clustering genes on the basis of their expression data, and highlighting, on the dendrogram showing the results, branches corresponding to a gene group having the same function and a gene group having an expression pattern similar to those of the gene group. Thus, the positions of these genes in the entire dendrogram can be comprehended.

However, it is difficult to predict a gene-gene interaction on the basis of the time-course graph and the pathway diagram. Furthermore, even if expression data of each gene is distributed into nodes of the pathway diagram, it is difficult to verify the pathway data and the experiments on the basis of the pathway and the expression data.

According to the method for displaying gene data in the above-mentioned related art, prediction of a gene-gene interaction is possible, but comparison between the pathway and experimental data is practically impossible. Therefore, the pathway and the experiments cannot be satisfactorily verified.

Accordingly, an embodiment of one aspect of the present invention can provide a method of analysis which can readily compare expression data and a pathway by using a computer, unlike a conventional analysis which subjectively compares or manually compares with certain criteria expression data and a pathway.

An embodiment of another aspect of the present invention can provide an expression data analysis method which includes a constructing process wherein a processor constructs expression data of a protein/gene other than a target protein/gene so as to fit in with a pathway on the basis of the target protein/gene expression data at an objective time-point/chemical; and a displaying process wherein the processor displays the constructed structure of the expression data constructed in the constructing process on a display and highlights protein/gene expression data fitting in with the pathway in the constructed structure of the expression data. Thus, in this embodiment, the expression data of the protein/gene other than the target protein/gene is constructed on the basis of the target protein/gene expression data in such a manner so that the protein/gene expression data fits in with the pathway; and the protein/gene expression data fitting in with the pathway in the constructed structure of the expression data is highlighted while the constructed structure of the expression data is displayed on the display. Therefore, the comparison of the pathway and the experimental values can be readily performed by viewing the display.

Protein(s)/gene(s) herein mean protein(s) or gene(s). Protein/gene corresponds to a node in the pathway. The node in the pathway is actually a gene or a protein at present, but possible nodes in a pathway will be included in the terms of protein/gene. Time-point(s)/chemical(s) mean time-point(s) or chemical(s).

An embodiment of another aspect of the present invention can provide an expression data analysis system which conducts the methods embodying the present invention described above.

An embodiment of another aspect of the present invention is a program of instructions executable by a computer to cause the computer to perform the steps of the above-described expression data analysis methods according to the present invention. The program may be embodied on or in a program storage device readable by the computer.

Reference will now be made, by way of example, to the accompanying drawings, in which:

FIG. 1 is a schematic explanatory diagram of a method according to an embodiment of the present invention.
FIG. 2 is one system configuration diagram used in a method according to an embodiment of the present invention.
FIG. 3 is an example of a hardware configuration according to an embodiment of the present invention.
FIG. 4 is a data structure and a process flow used in a method according to an embodiment of the present invention.
FIG. 5 is an explanatory diagram of an example for creating pathway logic control value data used in a method according to an embodiment of the present invention.
FIG. 6 is an enlarged diagram of (a), (b), (c), and (d) in FIG. 5.
FIG. 7 is an enlarged diagram of (e) and (f) in FIG. 5.

FIG. 8 is an explanatory diagram of an example for comparing pathway logic control data and experimental values (gene expression data) used in a method according to an embodiment of the present invention.

FIG. 9 is an enlarged diagram of (a), (b), and (c) in FIG. 8.

FIG. 10 is an enlarged diagram of (d), (e), and (f) in FIG. 8.

FIG. 11 is an example of a display of a pathway graph by a method according to an embodiment of the present invention.

FIG. 12 is a diagram clarifying FIG. 11.

FIG. 13 is an example of a display of a pathway graph at each time-point by a method using A as a starting point according to an embodiment of the present invention.

FIG. 14 is a reference matrix of types of edges, changes in expression level, and changes in active status according to an embodiment of the present invention.

FIG. 15 is an explanatory diagram of types of edges, control systems, and edge forms according to an embodiment of the present invention.

FIG. 16 is an explanatory diagram of an objective gene to be treated by a method according to an embodiment of the present invention.

FIG. 17 is a display by a conventional time-course graph.

FIG. 18 is a display by a conventional pathway diagram.

The present invention is operable in a large number of different ways. Therefore, the scope of the present invention should not be interpreted as being limited to the embodiments disclosed below.

In the embodiments, methods will be mainly described, but the present invention is also operable as a system or program used on a computer, which is common knowledge of one skilled in the art. Furthermore, the present invention is operable in embodiments of a hardware, software, or both software and hardware. The program can be recorded on any computer-readable medium such as a hard disk, CD-ROM, DVD-ROM, optical recording system, or magnetic recording system. The program can be also recorded on another computer over a network. The program may also be carried by a transmission medium such as a signal, instead of being stored by a recording medium.

In the embodiments, the present invention is described by using genes alone, but the present invention is applicable to protein in the same manner as the genes.

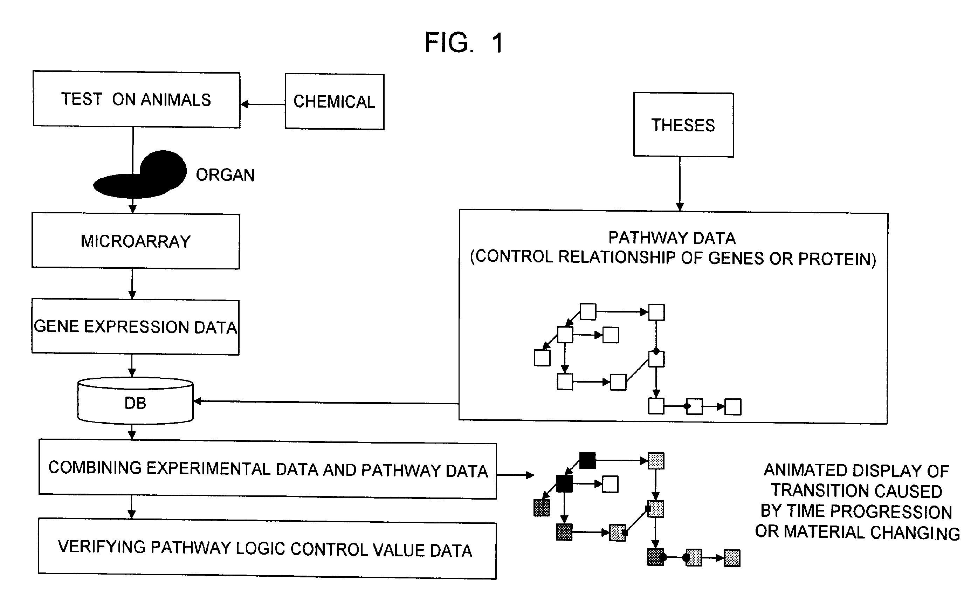

FIG. 1 is a schematic explanatory diagram of a method according to an embodiment of the present invention. Expression data can be obtained by, for example, publicly known gene expression analysis using microarray technology. In general, a probe DNA is prepared and is spotted on a slide glass. A target DNA is prepared and is conjugated with a fluorescent label. Then, hybridization and washing are performed, and a fluorescent signal is detected by a detector and converted into digital data with a computer. Thus, the expression data is obtained. The resulting gene expression data is stored in a database.

Pathways, which are interaction data between genes, can be obtained by collecting fragmentary pathways disclosed in papers. Some sites of outstanding databases (EcoCyc, MetaCyc, KEGG, TRANSPATH, CSNDB) are already accessible via the Internet. Pathways can be also obtained from such outstanding databases. These pathways are stored in the database.

A target gene is selected. The selected gene is a starting point of control. Data for specifying the gene may be input or selected. Alternatively, a node may be selected from a pathway diagram on a display. By the selection of the node, the corresponding gene can be specified. In this step, since the pathway diagram is used for only the selection of the node and is not required to be displayed by the method according to the present invention, a conventional display of the pathway diagram may be used. Only genes are used for description in this embodiment, but the present invention can be applied protein in the same manner.

A processor calculates the status of other genes on the pathway diagram on the basis of the pathway when the selected gene expression is up- controlled and down-controlled. The calculated results are defined as pathway simulation data. The processor reads out time-series gene expression data from the database by using all the nodes on the pathway as a key. Pathway mapping of the time-series gene expression data read out by the processor is performed. The processor searches gene expression data fitting in with the pathway simulation data from the time-series gene expression data and specifies the corresponding gene expression data in relation to each time-point of the selected gene.

The processor displays edges on the basis of the pathway on the display, and also distributes the gene expression data to the corresponding node positions for displaying them in the order according to the time-points. Furthermore, the processor distributes the present gene expression data to the corresponding node positions for displaying them. The processor highlights the present gene expression data specified from the distributed expression data. The processor calculates a score of the selected gene on the basis of the gene expression data specified at each time-point and the pathway simulation data. The scores calculated by the processor are summed.

Thus, the expression data of a gene corresponding to each node in the pathway diagram is displayed in the order according to the time-points, the expression data of a gene fitting in with the pathway is highlighted, and the scores of the constructed structure of the expression data are displayed. Therefore, the verification of the pathway and the experimental values can be readily performed. In the past, the gene expression data distributed to the pathway has been merely displayed. Consequently, it has been necessary to confirm whether the experimental data is along the pathway or not, which is troublesome. Furthermore, when an additional experiment is performed to add gene expression data, the experimental data must be further confirmed, which is also troublesome.

Using the method according to one embodiment of the present invention, the comparison of the pathway and the experimental values can be readily performed by viewing the display. For example, no highlighting in the constructed structure indicates that the pathway includes an error (the pathway itself is wrong, or the pathway itself is correct but the application of the pathway is wrong) or indicates that the experiment includes an error. Furthermore, by seeing the constructed structure including the highlighted protein/gene expression data, it can be understood what time course a gene takes to influence another gene. The above-mentioned processes can be performed to a plurality of objective time-points/chemicals, and the comparison of the pathway and experimental values can be also performed to the target protein/gene at a plurality of time-points/chemicals.

In an embodiment of the present invention, the protein/gene expression data at every time-point/chemical is further displayed on the display, and the protein/gene expression data fitting in with the pathway in the construction of the expression data is highlighted. Therefore, by seeing the display, the comparison of the pathway and the experimental values can be readily performed, in particular, in respect to the time course. For example, the highlighting of the protein/gene expression data at the same time-point/chemical indicates that a reaction from a controlling protein/gene till a controlled protein/gene is rapidly performed.

In an embodiment of the present invention, protein/gene expression data corresponding to each node in the pathway diagram is further displayed according to the time-points/chemicals, and the protein/gene expression data fitting in with the pathway from the protein/gene expression data constituting the construction constructed in the constructing process is highlighted. Therefore, the control relationship of protein/genes shown by the pathway can be clarified by the pathway diagram, and protein/gene expression data involved in the control relationship of the protein/gene is clarified by the highlight, in the protein/gene expression data. Thus, a user can very readily comprehend the pathway and experimental values.

In an embodiment of the present invention, the score is calculated on the basis of the number of the protein/gene expression data fitting in with the pathway, wherein the expression data is included in the protein/gene expression data constituting the construction of the expression data constituted in the constituting process. Therefore, a user can comprehend from the score how much the construction of the expression data fits in with the pathway. By calculating the score of every construction of the expression data of a plurality of objective time-points/chemicals, a user can determine which objective time-point/chemical mostly fits in with the pathway.

In an embodiment of the present invention, in addition to the number of the protein/gene expression data fitting in with a pathway, the difference between the objective time-point/chemical and the time-point/chemical of protein/gene expression data fitting in with the pathway is incorporated into the calculation of the score. Thus, a user can comprehend a combination of the expression data specifically relating to the target protein/gene.

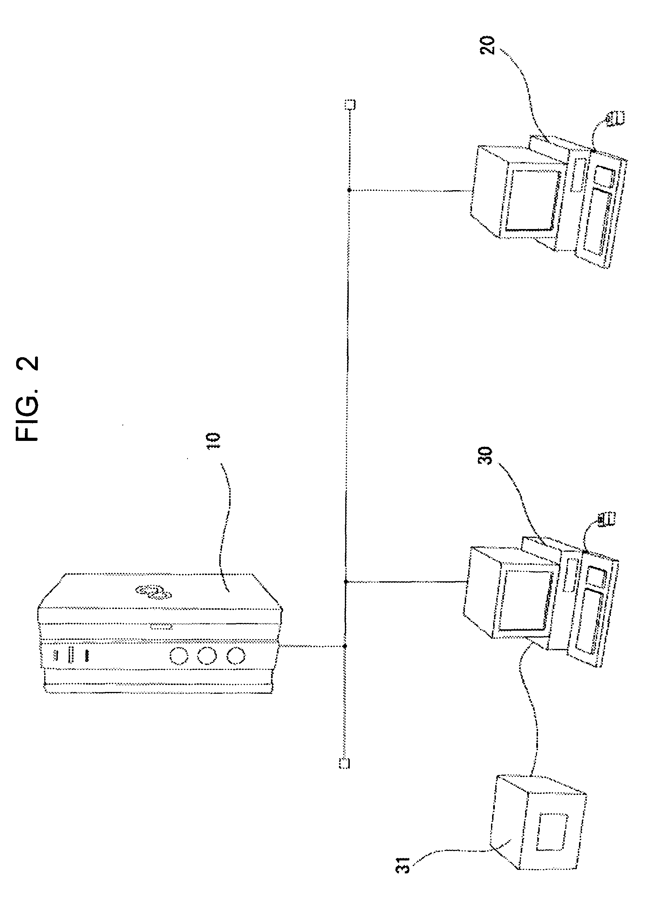

FIG. 2 is one system configuration diagram used in a method according to an embodiment of the present invention. In this embodiment, the system includes a host computer 10 which constructs a database recording various types of data; an analysis computer 20 which is a personal computer for reading out data from the database and for performing the most part of the method according to the present invention; and a detection computer 30 which is a personal computer for finding the expression data of each gene by being connected to a scanner with wired cable, detecting a fluorescent signal by a detector 31, and digitalizing the strength of the fluorescent signal.

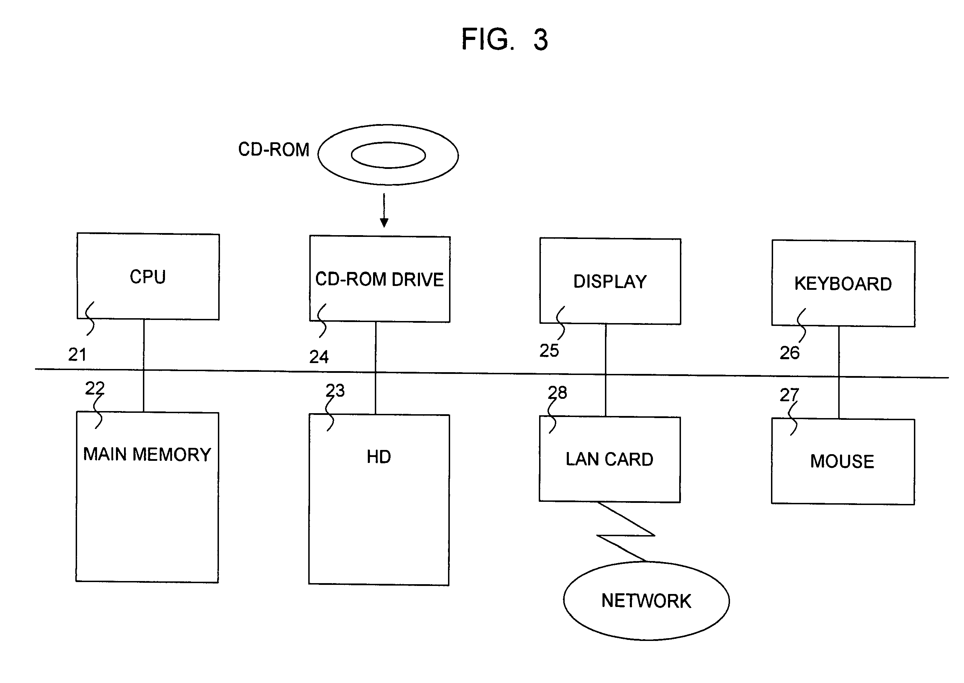

FIG. 3 is an example of a hardware configuration of the analysis computer 20. In this embodiment, the hardware is composed of a central processing unit (CPU) 21, main memory 22, a hard disk (HD) 23, a CD-ROM drive 24, a display 25, a keyboard 26, a mouse 27, and a LAN card 28, as in a general personal computer.

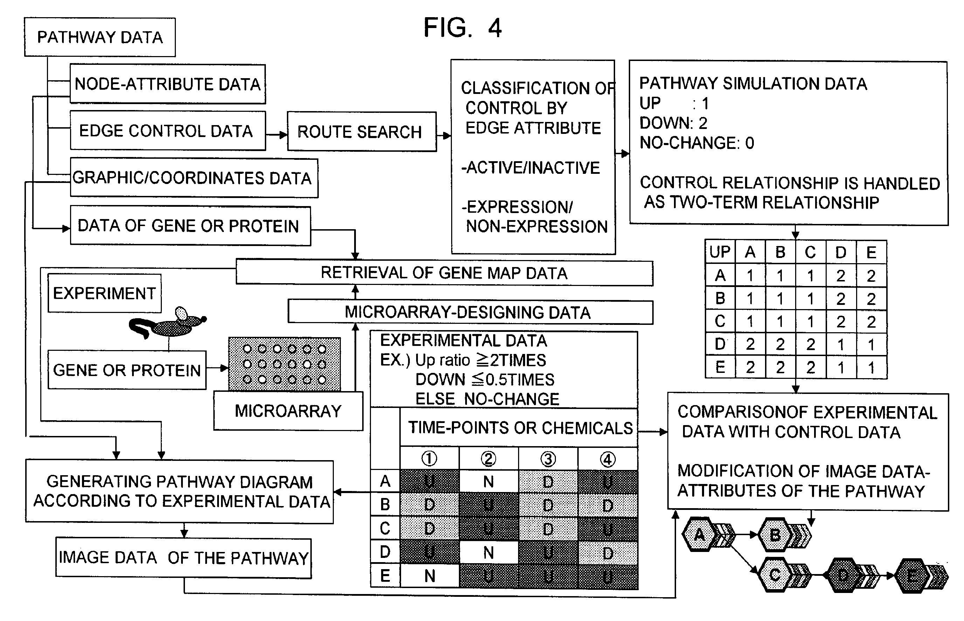

FIG. 4 is a data structure and a process flow used in the method according to an embodiment of the present invention. The pathway is composed of node-attribute data, edge control data, and graphic/coordinates data. The CPU 21 retrieves path-finding data (forward direction path) by route search on the basis of edge control data in the pathway. The pathway simulation data is retrieved on the basis of the path-finding data retrieved by the CPU 21. Expression data of a certain gene is obtained by the gene expression analysis using the microarray. When the microarray is used, the CPU 21 includes the microarray-designing data, i.e. which gene is aligned on which position on the microarray. The CPU 21 retrieves gene map data by combining the microarray-designing data and the node-attribute data of the pathway using genes as a key. The CPU 21 generates a pathway diagram from the graphic/coordinates data of the pathway, the gene map data, and experimental expression data. The pathway diagram can be displayed on the display 25 after color-coding according to the expression ratio. However, in the present invention, the CPU 21 further compares the experimental data and the pathway simulation data, and displays the gene expression data of a gene which status fits in with the pathway diagram by marking on the pathway diagram on the display 25. Examples of image data-attributes of the pathway include, as shown in the pathway diagram in lower right of FIG. 4, a node shape (hexagon), a node color, and node marking.

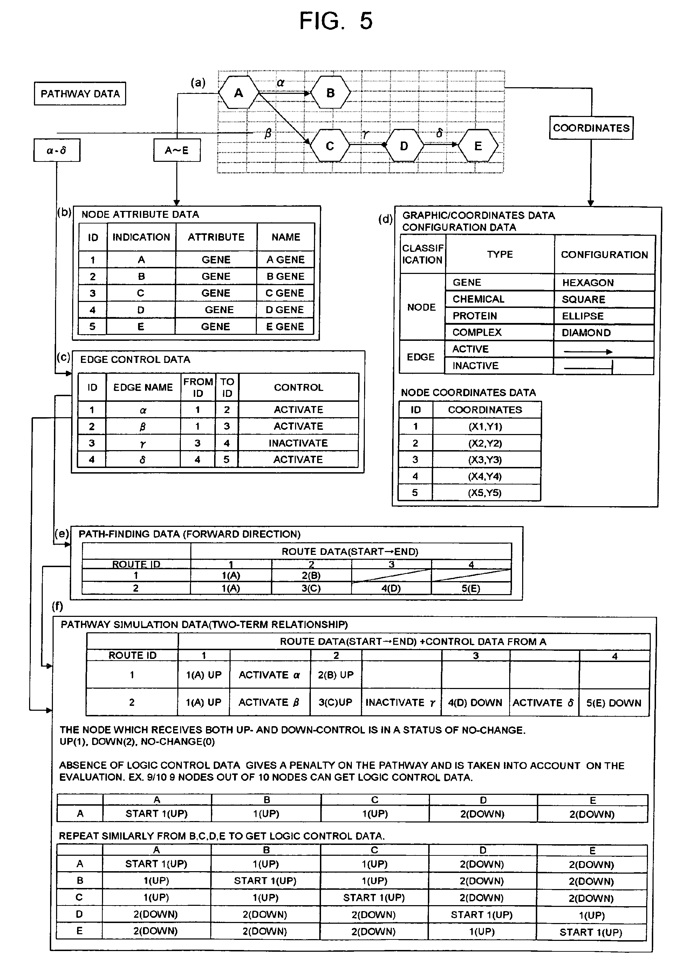

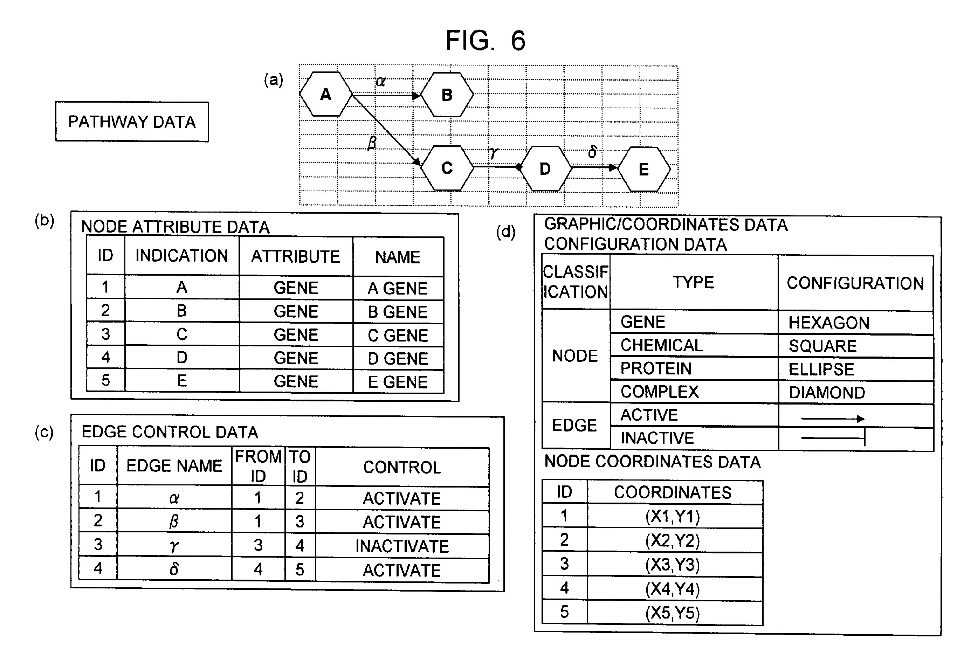

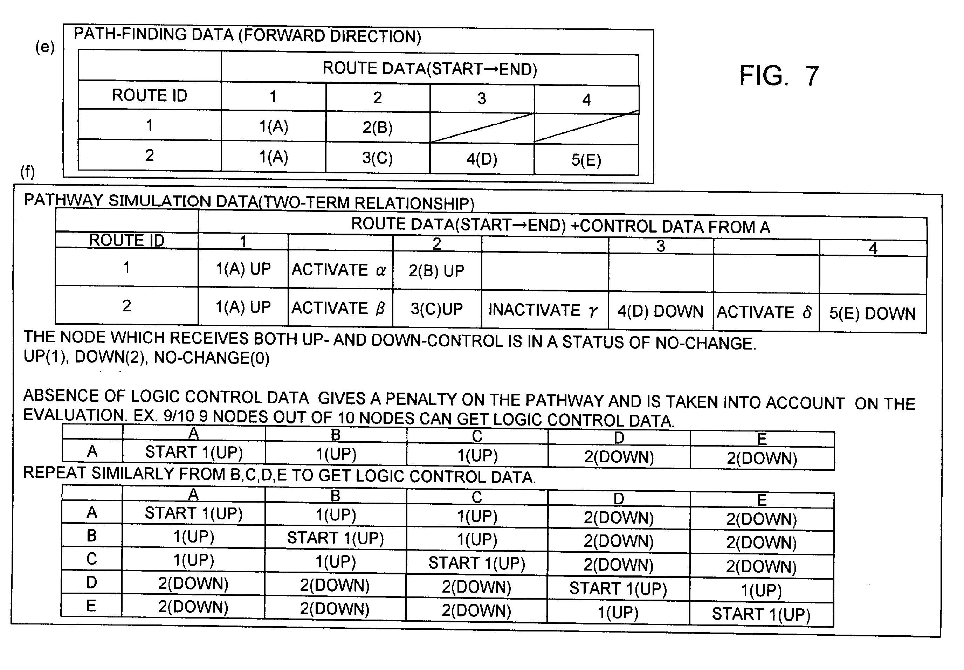

FIG. 5 is an explanatory diagram of a specific example for constructing pathway logic control value data used in a method according to an embodiment of the present invention. FIGs. 6 and 7 are enlarged diagrams of FIG. 5 for clarification. Elements of the node attribute data constituting the pathway include an ID, indication, attribute, and name. Elements of the edge control data constituting the pathway include an ID, name of edge, FromID, ToID, and control. Graphic/coordinates data constituting the pathway is composed of configuration data and node coordinates data. Elements of the configuration data include classification, type, and configuration. Elements of the node coordinates data include an ID and coordinates.

When the CPU 21 searches path-finding data from edge control data, various types of algorithms for finding a route can be used. In FIG. 5, since the pathway is simple for convenience in description, two routes can be readily found. When a large number of nodes and branches exist, an indefinitely large number of routes are found. In such a case, the longest route is preferably found (route-searching process) to suppress the number of routes in the path-finding data. The longest route can be found by various ways. For example, a way of simply following a reachable route, going back to a branch point from a dead end, and starting again from the branch point to search another route. After all possible routes are found, the longest route is selected (for example, the longest route is determined by the number of nodes existing between the starting point and the endpoint). Edges and controls corresponding between nodes of each route of path-finding data found by CPU 21 using A as a starting point are determined on the basis of the edge control data. Since the starting point is A, the routes of route ID 1 and route ID 2 are determined as shown in the top and middle tables of (f) in FIG. 5. Similarly, by starting from point B, C, D, or E, the routes of the each are determined to obtain logic control data shown in the bottom table of (f) in FIG. 5 (hypothetical protein/gene status-determining process). This logic control data indicates the status of other genes when a certain gene status is determined. For example, it is observed that when A gene is active, B gene and C gene are active and D gene and E gene are inactive. Here, the genes take two types of status, and it can be comprehended as a two-term relationship. Therefore, it is also understood that when A gene is inactive, B gene and C gene are inactive and D gene and E gene are active. Since these logic control data can be obtained from a pathway, they are preferably obtained from a database 10 in advance.

In an embodiment of the present invention, the longest route including the target protein/gene is searched in the protein/gene relating to the target protein/gene, the protein/gene status on the longest route is determined on the basis of the pathway. Then, the protein/gene status on routes other than the longest route is determined on the basis of the pathway and the previously determined protein/gene status, and protein/gene expression data is constructed so as to fit in with the determined protein/gene status. Therefore, the protein/gene status can be rapidly determined on the basis of the longest route.

In an embodiment of the present invention, the longest route including the target protein/gene is searched in the protein/gene relating to the target protein/gene, and the protein/gene status on the longest route is determined on the basis of the pathway. The protein/gene status on routes other than the longest route is not determined, and protein/ gene expression data is constructed so as to fit in with the determined protein/gene status on the route. Therefore, the protein/gene status to be determined and the protein/gene expression data to be compared are reduced to achieve a rapid response as a whole.

In an embodiment of the present invention, since the above-mentioned each process is performed to the range of only the target protein/gene and the target protein/gene-relating protein/gene out of the entire protein/gene, unnecessary calculation which is conducted to the protein/gene outside the range, can be avoided.

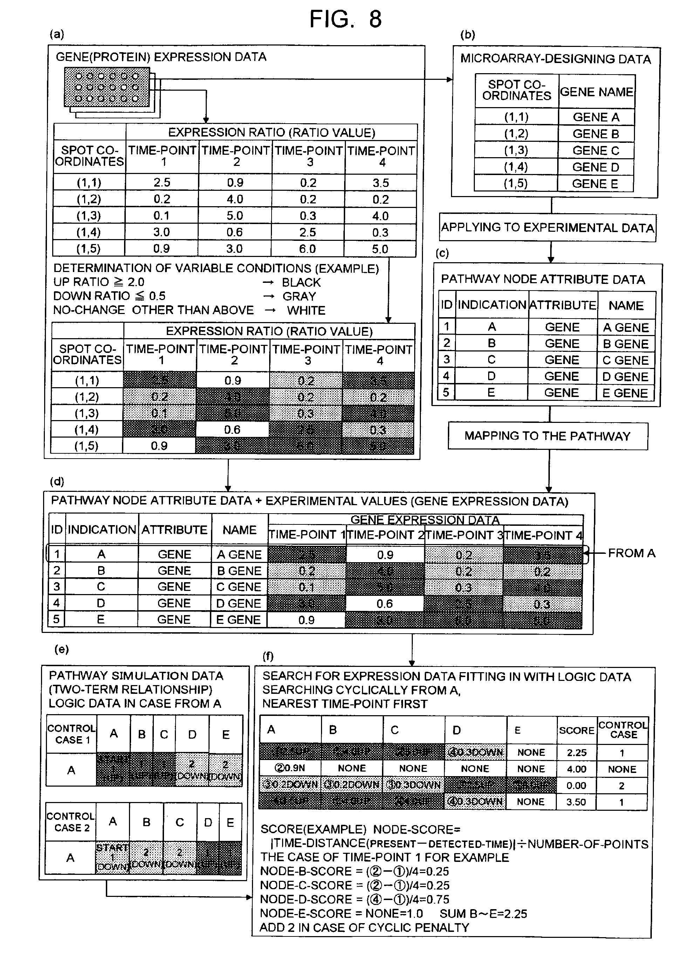

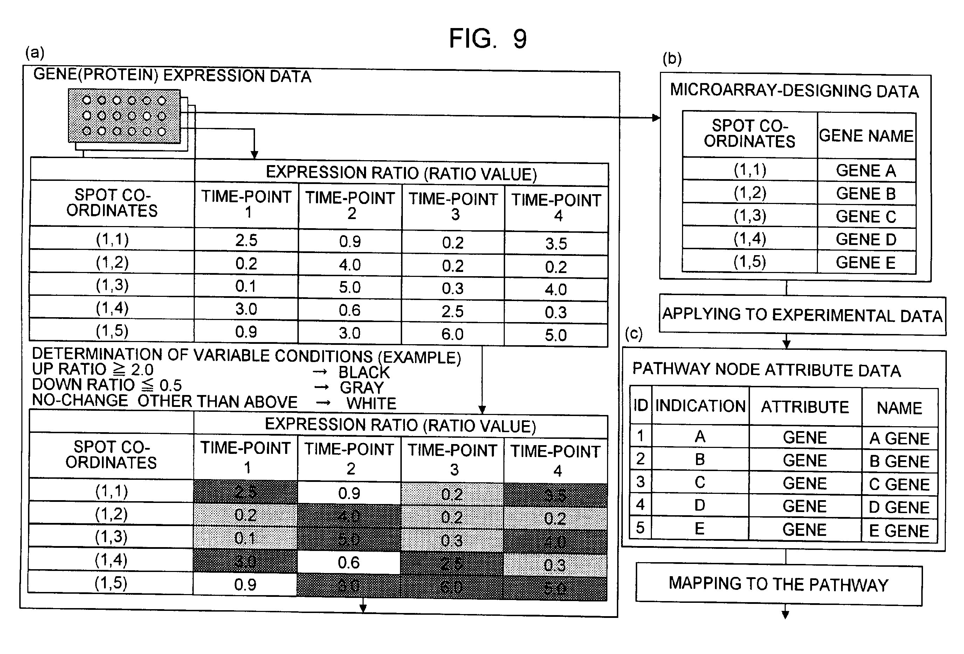

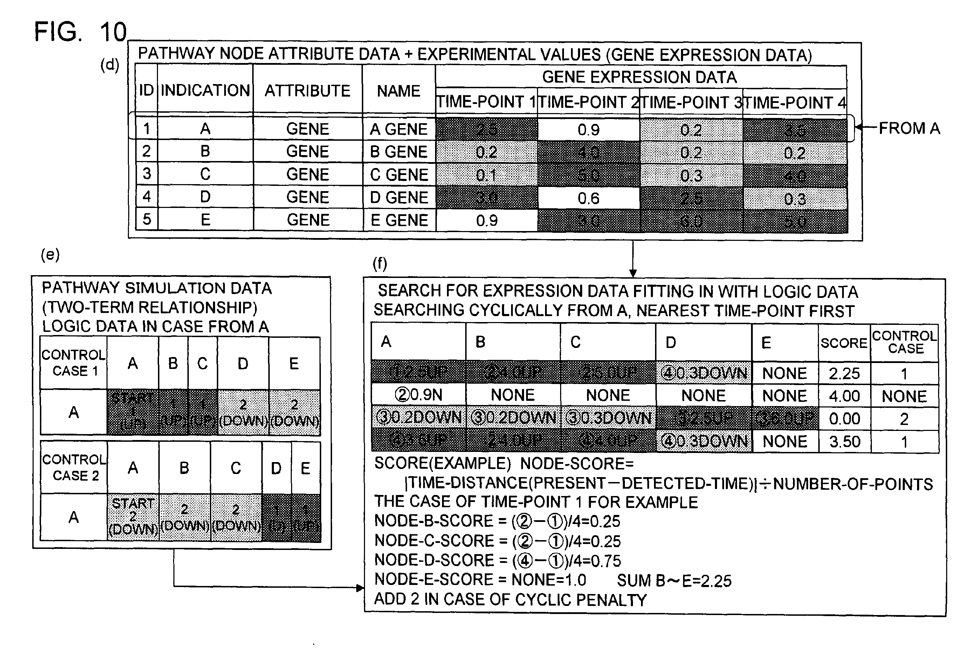

FIG. 8 is an explanatory diagram of an example for comparing pathway logic control data and experimental values (gene expression data) used in a method according to an embodiment of the present invention. FIGs. 9 and 10 are enlarged diagrams of FIG. 8 for clarification. The gene expression data is comprised of a spot coordinates, expression ratio at time-point 1, expression ratio at time-point 2, expression ratio at time-point 3, and expression ratio at time-point 4 (This is an example, so the data may include more time-points and expression data of a large number of genes). The bottom table of (a) in FIG. 8 is color-coded on the basis of determination of variable conditions, for explanatory convenience. As shown by (b) in FIG. 8, elements of the microarray-designing data include a spot coordinates and gene name. CPU 21 combines the gene expression data and the node attribute data on the basis of the microarray-designing data to make (d) in FIG. 8. The path simulation data which is already shown by (f) in FIG. 5 is shown by (e) in FIG. 8 again. When a target is A gene and the present time-point is time-point 1, expression ratios of B gene, C gene, D gene, and E gene at time-point 1 or later are constructed on the basis of the expression ratio of A gene at time-point 1 so as to fit in with path simulation data (constructing process). Accurately, a combination that a time-point of a controlled gene is previous to a time-point of a controlling gene is not created. This is because that the controlled gene status is determined by the control of the controlling gene. So, the time-point of the controlled gene generally is not previous to the time-point of the controlling gene, though the time-point of the controlled gene and the time-point of the controlling gene would be the same. If such a combination were possible, the above-mentioned combination, which is regarded not to be created, is created.

Similarly, the constructed structures for of the target gene A at time-point 2, time-point 3, and time-point 4 are constructed. Thus, the columns A to E in the table (f) in FIG. 8 are determined.

Scores of every constructed structure are calculated by the following formula (scoring process). When an expression ratio fitted in does not exist, the score is 1.0. When the present time-point and the detection time-point are the same, the score is 0. Namely, in addition to the number of the expression ratio fitted in, the difference between the objective time-point and the time-point of a gene other than the target gene is incorporated into the calculation of the score. Thus, a controlled gene, which is influenced at an early stage because of a small difference in the time-points, has a low score.

```
Node score = | distance of time-points (present time-

point detection time-point) | / number of time-point
```

According to this formula, the score of B gene is 0.25, the score of C gene is 0.25, the score of D gene is 0.75, and the score of E gene is 1.0, consequently, the score of the target A gene at time-point 1 is 2.25. Similarly, scores at time-point 2, time-point 3, and time-point 4 are calculated. Thus, the score column of (f) in FIG. 8 is determined. The last column of (f) in FIG. 8 is a control case, and it is shown which case of the top or bottom of (e) in FIG. 8 is applied or not applied when each combination is compared with the path simulation data.

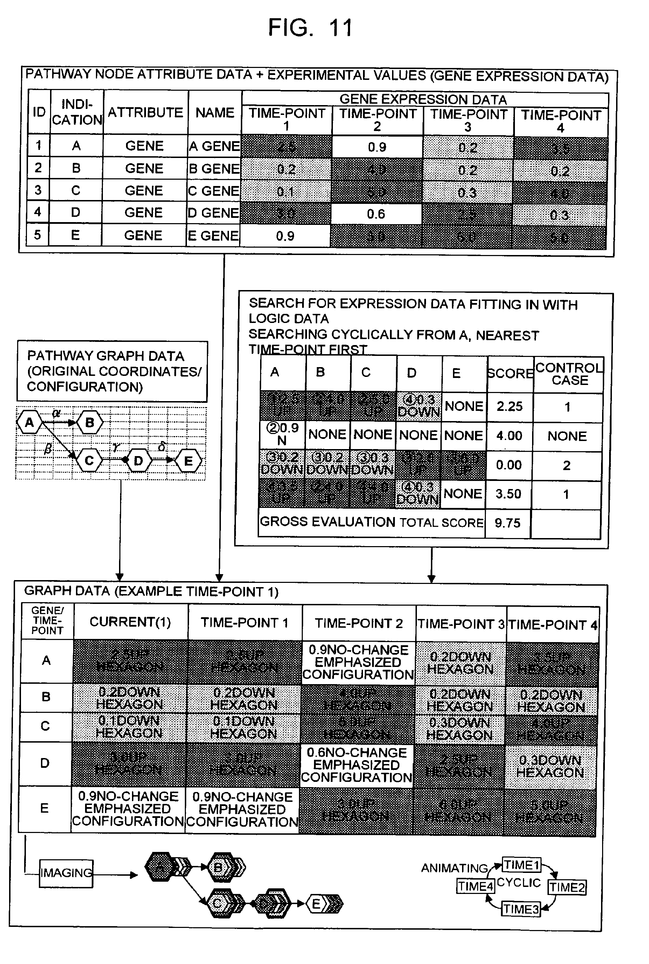

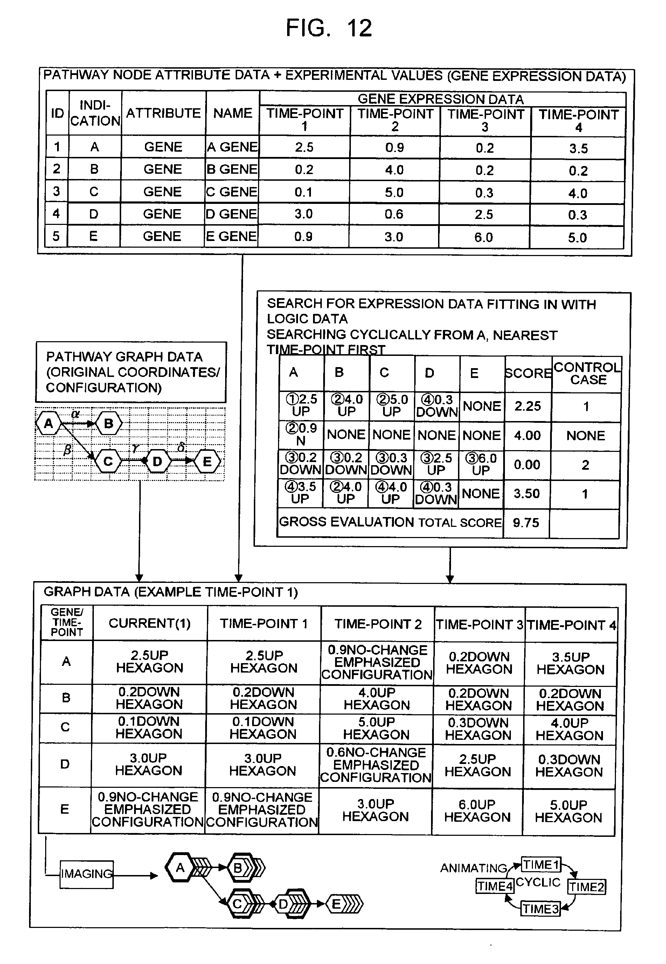

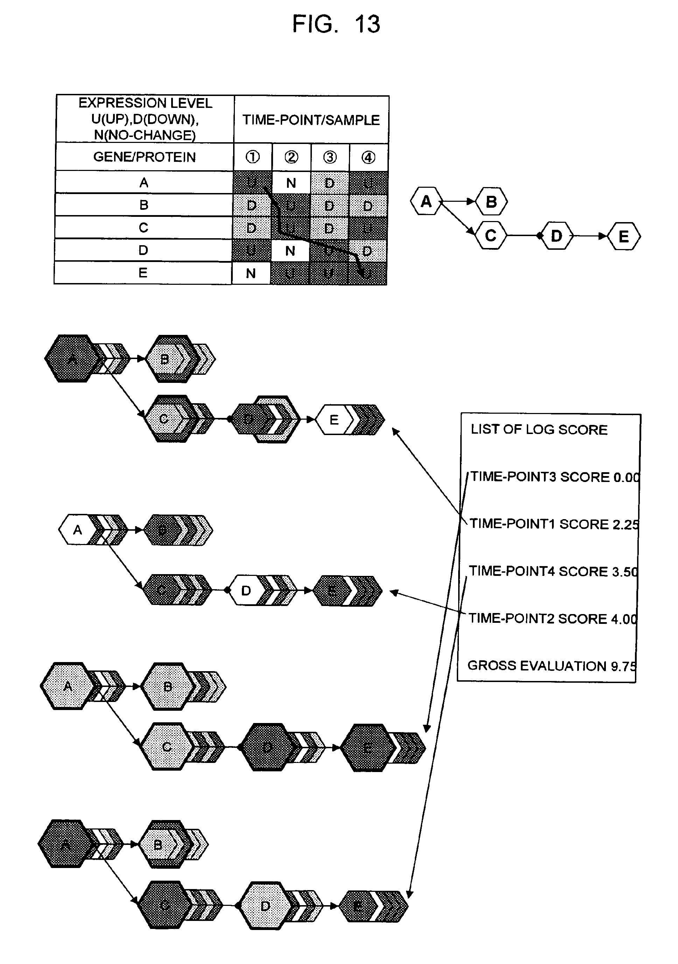

FIG. 11 is an example of a display of a pathway graph by a method according to an embodiment of the present invention. FIG. 12 is a diagram clarifying FIG. 11. Hexagons are color-coded according to the expression ratio range on the basis of every time-point at each node of a pathway diagram. The CPU 21 displays the hexagons piled with a small amount of displacement to each other from the hexagon of the time-point 4 to the hexagon of the time-point 1 in the order of time-points. The CPU 21 displays the present expression ratio of each node at each head of the nodes. In the combination specified in respect of the objective time-point, the hexagon relating to the specified expression ratio is drawn larger than other hexagons to distinguish from other hexagons and has the frame border being drawn with a color different from that of the inside the frame (displaying process). FIG. 13 is an example of a display of a pathway graph at each time-point by a method using A as a starting point according to an embodiment of the present invention.

'[Edge type and correspondence between change in expression level and change in active status]

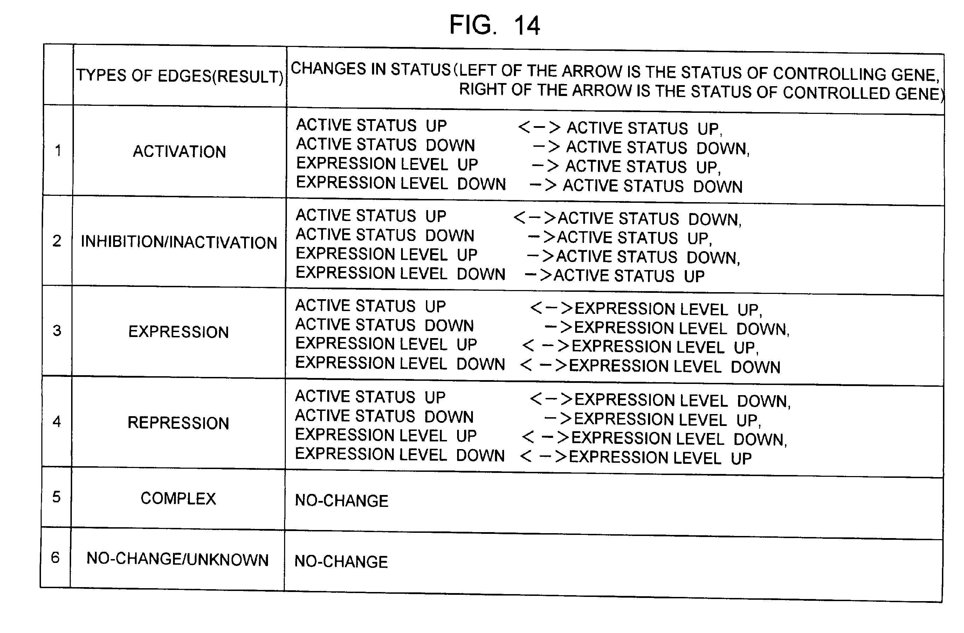

FIG. 14 is a reference matrix of types of edges, changes in expression level, and changes in active status.

- Possible gene (protein) status in expression level is "up", "down", and "no-change".
- Possible status in activity is "up", "down", and "no-change".
- Gene status is determined by edge type (result).
- A gene which is apart from the selected gene by the same distance and receives both up- and down-control is in a status of no-change.
- Status is determined on the basis of all control data of input pathways.

[Edge type, control system, and edge form]

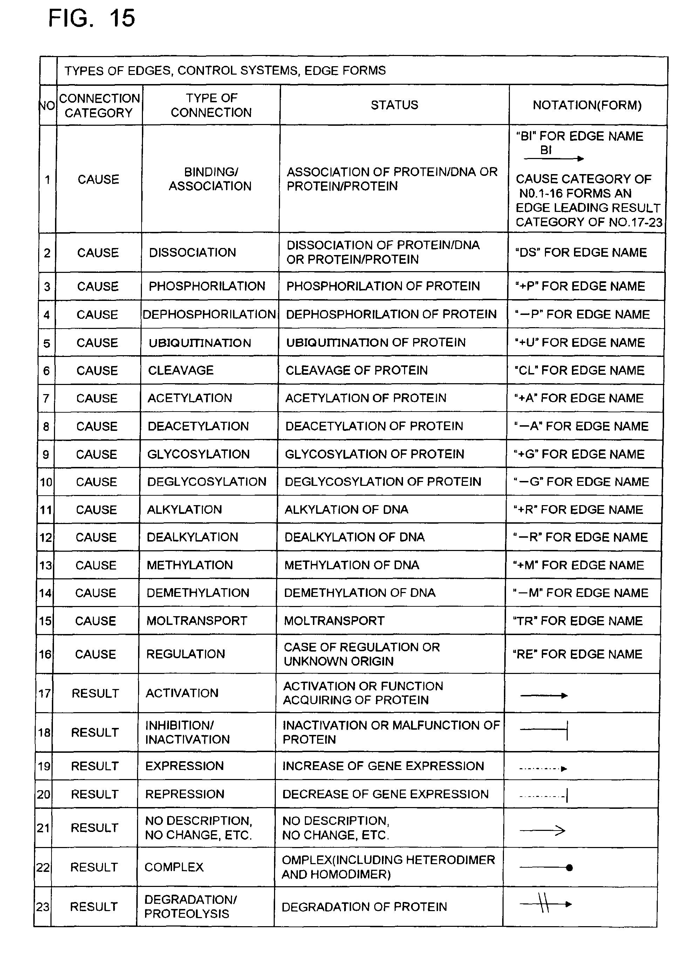

FIG. 15 is an explanatory diagram of types of edges, control systems, and edge forms. Since the binding of protein and a reaction such as phosphorylation generally tend to occur when the expression level is high, it is assumed that a high expression level enhances a reaction. Conversely, the reaction hardly occurs at a low expression level. From this assumption, when the expression level of a controlling gene is "up" and the type of the edge is activation, it is defined the active status of the controlled gene is "up".

[Determination of objective gene]

In the case that a user determines a target gene, a gene (B and C in the case of (a) in FIG. 5) controlled by the target gene (A in the case of (a) in FIG. 5) is defined as a directly controlled gene, and a gene (D in the case of (a) in FIG. 5) controlled by the controlled gene is defined as an indirectly controlled gene. A gene (E in the case of (a) in FIG. 5) directly or indirectly controlled by the directly controlled gene or the indirectly controlled gene is also defined as an indirectly controlled gene, hereinafter. Namely, genes which are traced back to the target gene are defined as directly or indirectly controlled genes. In such a case, only the target gene, the directly controlled gene, and the indirectly controlled gene receive the constructing process, the displaying process, and the scoring process; and other genes do not receive these processes. Thus, the display can be rapidly performed without any unnecessary process. Here, only the expression data of the other genes is displayed in the order according to the time-points at nodes on the pathway diagram. Furthermore, it is possible that when a target gene is designated by a user, the designated target gene, the directly controlled gene, and indirectly controlled gene are presented so that the user can select a necessary or unnecessary gene. Then, the constructing process, the displaying process, and the scoring process are performed to only the objective gene to be verified by the user. Thus, the processes are rapidly performed and an unnecessary display is also avoided, so the user can perform the verification more smoothly.

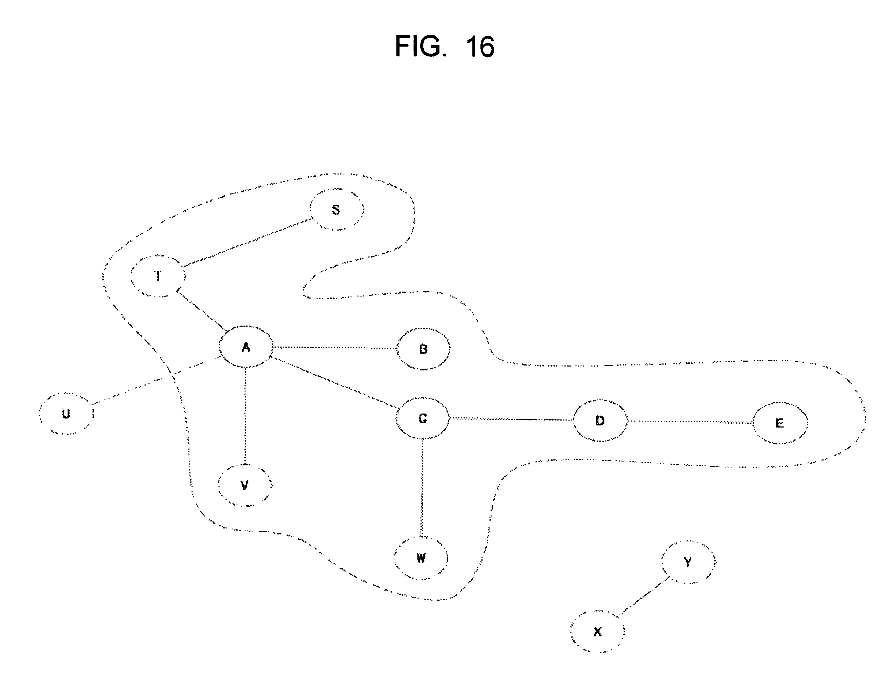

Furthermore, when the constructing process, the displaying process, and the scoring process are performed to only a gene relating to the target gene and these processes are not performed to other genes, unnecessary processes are omitted to rapidly perform the display. Noises caused by unrelated data can be also deleted from the score values. The gene relating to the target gene includes a gene directly or indirectly controlled by the target gene and a gene directly or indirectly controlling the target gene. FIG. 16 shows the nodes A, B, C, D, and E which are used in the embodiment, and nodes S, T, U, V, W, X, and Y. In this case, the nodes relating to the target node are nodes S, T, V, W, B, C, D, and E. Nodes U, X, and Y are not included. Node U has a relationship to A, but the relationship in this situation is omitted from the objective. As shown in FIGS. 14 and 15, a plurality of relationships are possible, a user may designate an objective relationship. The dashed line in FIG. 16 clearly specifies the nodes of the target gene and the gene relating to the target gene. With such a dashed line on a display of the pathway diagram, a user can readily comprehend the relationship.

[Search for route including objective gene]

A user can also designate an objective gene for verification in addition to the target gene. When the route-searching is performed by this designation, routes including the target gene and the designated gene are searched. The status of genes on the routes is determined at first, and then the status of genes outside the routes is determined on the basis of the status which is previously determined. Since the longest route including the protein/gene designated by the user is searched, the protein/gene status is rapidly determined on the basis of the protein/gene specifically comprehended by the user. Thus, the status of genes other than the target and designated genes are determined on the basis of the status of the designated gene. Therefore, the verification can be performed concentrating on the gene designated by the user.

[Display of objective time-point designated by user]

The switching of a display at an objective time-point designated by a user as an objective for verification can be achieved by that the user designates the objective time-point and a marking display and a score display of a pathway diagram relating to the designated objective time-point are displayed. In this case, all time-points may be previously required as the objective time-points or may be required depending to need. For example, a format is possible that when a circulation of the time-points in FIG. 11 is displayed on a display, the user selects a time-point and the displaying process is performed for the time-point designated by the user as the objective time-point.

Therefore, protein/gene expression data fitting in with a pathway at each time-point/chemical is highlighted according to the designation of a user. Thus, a change in influence of a protein/gene to another protein/gene can be comprehended.

[Animation by automatically and sequentially specifying objective time-points]

A user does not specify the objective time-point, alternatively, a format that the objective time-points are automatically specified at predetermined intervals is possible. In this case, the marking display and score display of the pathway diagram are sequentially drawn to achieve animation. Therefore, protein/gene expression data fitting in with a pathway at each time-point/chemical is sequentially highlighted at predetermined intervals of time. Thus, a change in influence of a protein/gene to another protein/gene can be further readily comprehended. By the time-series animation, the user can readily perform the verification.

[Display by specifying score]

When a user specifies a score, a marking display can be performed in respect to the constructed structure of the expression data corresponding to the specified score. The verification is readily performed by that the user selects the score from the highest value in the order of the scores and sees the pathway diagrams.

[Chemical instead of time-point]

The embodiments above are relating to the time-points. However, a format for chemicals instead of the time-points is possible; expression data of every chemical added to a gene is collected and is stored in a database; and processes for the marking display and score-calculating are performed to every objective chemical. Furthermore, expression data of every chemical at every time-point of the chemicals is collected and is stored in the database; and processes for the marking display and score-calculating can be performed to every chemical at every time-point so that both the objective time-points and objective chemicals are operable.

[Log-output in batch]

In the embodiments above, A gene is used as a target gene. However, the constructing process and the scoring process can be performed in batch mode to all genes of A to E to display log data on a display. Thus, for example, by selecting a constructed structure of expression data having a high score, the verification of the pathway and experimental values can be readily performed.

While the present invention has been described with respect to the above-mentioned embodiments, the technical scope of the present invention is not limited to the specifics described in those embodiments. Various changes or modification can be made to the embodiments. It will be apparent from the claims and the summary of the invention that embodiments including those changes and modifications are also included in the technical scope of the present invention.

**Claims**

**1.** Method for analyzing expression data comprising:

a constructing step of constructing expression data of a first set of protein or genes other than a target protein or gene to fit in with a pathway as much as possible according to expression data of the target protein or gene at an objective time-point or against an objective chemical; and

a displaying step of displaying a constructed structure constructed in said constructing step, and highlighting expression data of a second set of protein or genes fitting in with the pathway, the second set of protein or genes being included in the first set of protein or genes, the expression data highlighted being included in the constructed structure.

**2.** Method for analyzing expression data comprising:

a constructing step of constructing expression data of a first set of protein or genes other than a target protein or gene to fit in with a pathway as much as possible according to expression data of the target protein or gene at an objective time-point or against an objective chemical; and

a displaying step of displaying expression data of every protein or gene in the first set of protein or genes at each time-point or against each chemical, and highlighting expression data of a second set of protein or genes fitting in with the pathway, the second set of protein or genes being included in the first set of protein or genes, the expression data highlighted being included in a constructed structure constructed in said constructing step.

**3.** Method for analyzing expression data comprising:

a constructing step of constructing expression data of a first set of protein or genes other than a target protein or gene to fit in with a pathway as much as possible according to expression data of the target protein or gene at an objective time-point or against an objective chemical; and

a displaying step of displaying a node of the pathway and expression data corresponding to the node in order of time-points or chemicals according to the pathway and the expression data of the first set of protein or genes, and highlighting expression data of a second set of protein or genes fitting in with the pathway, the second set of protein or genes being included in the first set of protein or genes, the expression data highlighted being included in a constructed structure constructed in said constructing step.

**4.** Method for analyzing expression data comprising:

a constructing step of constructing expression data of a first set of protein or genes other than a target protein or gene to fit in with a pathway as much as possible according to expression data of the target protein or gene at an objective time-point or against an objective chemical; and

a displaying step of displaying a node of the pathway and expression data corresponding to the node in order of time-points or chemicals according to the pathway and the expression data of the first set of protein or genes, and highlighting expression data of a second set of protein or genes fitting in with the pathway, the second set of protein or genes being included in the first set of protein or genes, the expression data highlighted being included in a constructed structure constructed in said constructing step, the expression data displayed being at a time-point designated by a user or being against a chemical designated by a user.

**5.** Method for analyzing expression data comprising:

a constructing step of constructing expression data of a first set of protein or genes other than a target protein or gene to fit in with a pathway as much as possible according to expression data of the target protein or gene at an objective time-point or against an objective chemical; and

a displaying step of displaying a node of the pathway and expression data corresponding to the node in order of time-points or chemicals according to the pathway and the expression data of the first set of protein or genes, and highlighting expression data of a second set of protein or genes fitting in with the pathway, the second set of protein or genes being included in the first set of protein or genes, the expression data highlighted being included in a constructed structure constructed in said constructing step, the expression data displayed being at a time-point selected automatically and sequentially at predetermined intervals of time or being against a chemical selected automatically and sequentially at predetermined intervals of time.

**6.** Method for analyzing expression data comprising:

a constructing step of constructing expression data of a first set of protein or genes other than a target protein or gene to fit in with a pathway as much as possible according to expression data of the target protein or gene at an objective time-point or against an objective chemical; and

a scoring step of calculating a score according to a number of expression data of a second set of protein or genes fitting in with the pathway, the second set of protein or genes being included in the first set of protein or genes, the expression data of the second set of protein or genes being included in a constructed structure constructed in said constructing step.

**7.** The method of claim 6, wherein

the score is calculated in said scoring step, taking into account a difference between a time-point or chemical of the expression data of the second set of protein or genes fitting in with the pathway and the objective time-point or chemical of the target protein or gene.

**8.** The method of claim 6 or 7, further comprising:

a displaying step of displaying a constructed structure constructed in said constructing step and the score calculated in said scoring step correspondingly, displaying a node of the pathway and expression data corresponding to the node in order of time-points or chemicals according to the pathway and the expression data of the first set of protein or genes, and highlighting expression data of a second set of protein or genes fitting in with the pathway, the second set of protein or genes being included in the first set of protein or genes, the expression data highlighted being included in a constructed structure corresponding to a score designated by a user, the score designated by the user being included the score displayed in said displaying step, the constructed structure corresponding to the score designated by the user being included in the constructed structure constructed in said constructing step, wherein

said constructing step and said scoring step are executed at a plurality of time-points or against a plurality of chemicals, each time-point or each chemical being regarded as the objective time-point or the objective chemical.

**9.** The method of claim 1, further comprising:

a route-searching step of searching a longest route among routes between the target protein or gene and protein or a gene of a third set of protein or genes relating to the target protein or gene, the third set of protein or genes being included in the first set of protein or genes;

a hypothetical status determining step of determining status of a fourth set of protein or genes on the longest route according to the pathway, and determining status of a fifth set of protein or genes other than the fourth set of protein or genes on the longest route according to the pathway and the status of protein or genes determined already, the fourth set of protein or genes being included in the first set of protein or genes, the fifth set of protein or genes being included in the first set of protein or genes, wherein

the expression data of the first set of protein or genes is constructed to fit in with the status of protein or genes determined in said hypothetical status determining step instead of the pathway as much as possible in said constructing step.

**10.** The method of claim 1, further comprising:

a route-searching step of searching a longest route among routes between the target protein or gene and protein or a gene of a third set of protein or genes relating to the target protein or gene, the third set of protein or genes being included in the first set of protein or genes; and

a hypothetical status determining step of determining status of a fourth set of protein or genes on the longest route according to the pathway, the fourth set of protein or genes being included in the first set of protein or genes, wherein

the expression data of the first set of protein or genes is constructed to fit in with the status of protein or genes determined in said hypothetical status determining step instead of the pathway as much as possible in said constructing step.

**11.** The method of claim 9 or 10, wherein

the longest route searched in said route-searching step includes protein or a gene designated by a user, the protein or gene designated by the user being included in the first set of protein or genes.

**12.** The method of claim 1, further comprising:

a relating object specifying step of specifying a third set of protein or genes relating to the target protein or gene, the third set of protein or genes being included in the first set of protein or genes, wherein

said steps other than said relating object specifying step are executed for the third set of protein or genes and the target protein or gene.

**13.** A method as claimed in any preceding claim, carried out by electronic data processing means.

**14.** System for analyzing expression data comprising:

a constructing unit for constructing expression data of a first set of protein or genes other than a target protein or gene to fit in with a pathway as much as possible according to expression data of the target protein or gene at an objective time-point or against an objective chemical; and

a displaying unit for displaying a node of the pathway and expression data corresponding to the node in order of time-points or chemicals according to the pathway and the expression data of the first set of protein or genes, and highlighting expression data of a second set of protein or genes fitting in with the pathway, the second set of protein or genes being included in the first set of protein or genes, the expression data highlighted being included in a constructed structure constructed by said constructing unit, the expression data displayed being at a time-point selected automatically and sequentially at predetermined intervals of time or being against a chemical selected automatically and sequentially at predetermined intervals of time.

**15.** System for analyzing expression data comprising:

a constructing unit for constructing expression data of a first set of protein or genes other than a target protein or gene to fit in with a pathway as much as possible according to expression data of the target protein or gene at an objective time-point or against an objective chemical; and

a scoring unit for calculating a score according to a number of expression data of a second set of protein or genes fitting in with the pathway, the second set of protein or genes being included in the first set of protein or genes, the expression data of the second set of protein or genes being included in a constructed structure constructed by said constructing unit.

**16.** The system of claim 14 or 15, further comprising:

a route-searching unit for searching a longest route among routes between the target protein or gene and protein or a gene of a third set of protein or genes relating to the target protein or gene, the third set of protein or genes being included in the first set of protein or genes; and

a hypothetical status determining unit for determining status of a fourth set of protein or genes on the longest route according to the pathway, and determining status of a fifth set of protein or genes other than the fourth set of protein or genes according to the pathway and the status of protein or genes determined already, the fourth set of protein or genes being included in the first set of protein or genes, the fifth set of protein or genes being included in the first set of protein or genes, wherein

said constructing unit constructs expression data of the first set of protein or genes to fit in with the status of protein or genes determined by said hypothetical status determining unit instead of the pathway as much as possible.

17. A computer program which, when executed by a computer, causes the computer to carry out a method of analyzing expression data, said program comprising:

a constructing portion which causes the computer to construct expression data of a first set of protein or genes other than a target protein or gene to fit in with a pathway as much as possible according to expression data of the target protein or gene at an objective time-point or against an objective chemical; and

a displaying portion which causes the computer to display a node of the pathway and expression data corresponding to the node in order of time-points or chemicals according to the pathway and expression data of the first set of protein or genes, and to highlight expression data of a second set of protein or genes fitting in with the pathway, the second set of protein or genes being included in the first set of protein or genes, the expression data highlighted being included in a constructed structure constructed in said constructing step, the expression data displayed being at a time-point selected automatically and sequentially at predetermined intervals of time or being against a chemical selected automatically and sequentially at predetermined intervals of time.

18. A computer program which, when executed by a computer, causes the computer to carry out a method of analyzing expression data, said program comprising:

a constructing portion which causes the computer to construct expression data of a first set of protein or genes other than a target protein or gene to fit in with a pathway as much as possible according to expression data of the target protein or gene at an objective time-point or against an objective chemical; and

a scoring portion which causes the computer to calculate a score according to a number of expression data of a second set of protein or genes fitting in with the pathway, the second set of protein or genes being included in the first set of protein or genes, the expression data of the second set of protein or genes being included in a constructed structure constructed in said constructing step.

19. The program of claim 17 or 18, further comprising:

a route-searching portion which causes the computer to search a longest route among routes between the target protein or gene and protein or a gene of a third set of protein or genes relating to the target protein or gene, the third set of protein or genes being included in the first set of protein or genes; and

a hypothetical status determining portion which causes the computer to determine status of a fourth set of protein or genes on the longest route according to the pathway, and to determine status of a fifth set of protein or genes other than the fourth set of protein or genes according to the pathway and the status of protein or a gene determined already, the fourth set of protein or genes being included in the first set of protein or genes, the fifth set of protein or genes being included in the first set of protein or genes, wherein

the expression data of the first set of protein or genes is constructed to fit in with the status of protein or genes determined in said hypothetical status determining step instead of the pathway as much as possible in said constructing step.

20. System for analyzing expression data comprising:

a constructing unit for constructing expression data of a first set of protein or genes other than a target protein or gene to fit in with a pathway as much as possible according to expression data of the target protein or gene at a time-point or against a chemical; and

a displaying unit for displaying a constructed structure constructed by said constructing unit, and highlighting expression data of a second set of protein or genes fitting in with the pathway, the second set of protein or genes being included in the first set of protein or genes, the expression data highlighted being included in the constructed structure.

**21.** A computer program which, when executed by a computer, causes the computer to carry out a method of analyzing expression data, said program comprising:

a constructing portion which causes the computer to construct expression data of a first set of protein or genes other than a target protein or gene to fit in with a pathway as much as possible according to expression data of the target protein or gene at a time-point or against a chemical; and

a displaying portion which causes the computer to display a constructed structure constructed in said constructing step, and to highlight expression data of a second set of protein or genes fitting in with the pathway, the second set of protein or genes being included in the first set of protein or genes, the expression data highlighted being included in the constructed structure.

**22.** A computer program which, when executed by a computer, causes the computer to carry out the method of any one of claims 1 to 12.

**23.** A computer program as claimed in any one of claims 17 to 19, 21 and 22, carried by a carrier medium.

**24.** A computer program as claimed in claim 23, wherein said carrier medium is a recording medium.

**25.** A computer program as claimed in claim 23, wherein said carrier medium is a transmission medium.

# FIG. 1

ANIMATED DISPLAY OF
TRANSITION CAUSED
BY TIME PROGRESSION
OR MATERIAL CHANGING

FIG. 2

EP 1 742 161 A1

# FIG. 3

CD-ROM

CPU

CD-ROM DRIVE

DISPLAY

KEYBOARD

21

24

25

26

22

23

28

27

MAIN MEMORY

HD

LAN CARD

MOUSE

NETWORK

EP 1 742 161 A1

## FIG. 4

PATHWAY DATA

NODE-ATTRIBUTE DATA

EDGE CONTROL DATA → ROUTE SEARCH

GRAPHIC/COORDINATES DATA

DATA OF GENE OR PROTEIN

CLASSIFICATION OF CONTROL BY EDGE ATTRIBUTE

-ACTIVE/INACTIVE

-EXPRESSION/ NON-EXPRESSION

PATHWAY SIMULATION DATA
UP : 1
DOWN: 2
NO-CHANGE: 0

CONTROL RELATIONSHIP IS HANDLED AS TWO-TERM RELATIONSHIP

RETRIEVAL OF GENE MAP DATA

EXPERIMENT

MICROARRAY-DESIGNING DATA

GENE OR PROTEIN

MICROARRAY

| UP | A | B | C | D | E |
|----|---|---|---|---|---|
| A | 1 | 1 | 1 | 2 | 2 |
| B | 1 | 1 | 1 | 2 | 2 |
| C | 1 | 1 | 1 | 2 | 2 |
| D | 2 | 2 | 2 | 1 | 1 |
| E | 2 | 2 | 2 | 1 | 1 |

EXPERIMENTAL DATA
EX.) Up ratio ≧2TIMES
DOWN ≦0.5TIMES
ELSE NO-CHANGE

| | TIME-POINTS OR CHEMICALS | | | |
|---|---|---|---|---|
| | ① | ② | ③ | ④ |
| A | U | N | D | U |
| B | D | U | D | D |
| C | D | U | D | U |
| D | U | N | U | D |
| E | N | U | U | U |

COMPARISON OF EXPERIMENTAL DATA WITH CONTROL DATA

MODIFICATION OF IMAGE DATA-ATTRIBUTES OF THE PATHWAY

GENERATING PATHWAY DIAGRAM ACCORDING TO EXPERIMENTAL DATA

IMAGE DATA OF THE PATHWAY

# FIG. 5

PATHWAY DATA

(a)

COORDINATES

α-δ   A~E

Nodes: A → B (α), A → C (β), C → D (γ), D → E (δ)

**(b) NODE ATTRIBUTE DATA**

| ID | INDICATION | ATTRIBUTE | NAME |
|----|-----------|-----------|------|
| 1 | A | GENE | A GENE |
| 2 | B | GENE | B GENE |
| 3 | C | GENE | C GENE |
| 4 | D | GENE | D GENE |
| 5 | E | GENE | E GENE |

**(c) EDGE CONTROL DATA**

| ID | EDGE NAME | FROM ID | TO ID | CONTROL |
|----|-----------|---------|-------|---------|
| 1 | α | 1 | 2 | ACTIVATE |
| 2 | β | 1 | 3 | ACTIVATE |
| 3 | γ | 3 | 4 | INACTIVATE |
| 4 | δ | 4 | 5 | ACTIVATE |

**(d) GRAPHIC/COORDINATES DATA CONFIGURATION DATA**

| CLASSIFICATION | TYPE | CONFIGURATION |
|----------------|------|---------------|
| NODE | GENE | HEXAGON |
| NODE | CHEMICAL | SQUARE |
| NODE | PROTEIN | ELLIPSE |
| NODE | COMPLEX | DIAMOND |
| EDGE | ACTIVE | ──────► |
| EDGE | INACTIVE | ──────┤ |

**NODE COORDINATES DATA**

| ID | COORDINATES |
|----|-------------|
| 1 | (X1,Y1) |
| 2 | (X2,Y2) |
| 3 | (X3,Y3) |
| 4 | (X4,Y4) |
| 5 | (X5,Y5) |

**(e) PATH-FINDING DATA (FORWARD DIRECTION)**

| ROUTE ID | ROUTE DATA(START→END) | | | |
|----------|------|------|------|------|
| | 1 | 2 | 3 | 4 |
| 1 | 1(A) | 2(B) | | |
| 2 | 1(A) | 3(C) | 4(D) | 5(E) |

**(f) PATHWAY SIMULATION DATA(TWO-TERM RELATIONSHIP)**

| ROUTE ID | ROUTE DATA(START→END) +CONTROL DATA FROM A | | | | | | |
|----------|---|---|---|---|---|---|---|
| | 1 | | 2 | | 3 | | 4 |
| 1 | 1(A) UP | ACTIVATE α | 2(B) UP | | | | |
| 2 | 1(A) UP | ACTIVATE β | 3(C)UP | INACTIVATE γ | 4(D) DOWN | ACTIVATE δ | 5(E) DOWN |

THE NODE WHICH RECEIVES BOTH UP- AND DOWN-CONTROL IS IN A STATUS OF NO-CHANGE.
UP(1), DOWN(2), NO-CHANGE(0)

ABSENCE OF LOGIC CONTROL DATA GIVES A PENALTY ON THE PATHWAY AND IS TAKEN INTO ACCOUNT ON THE EVALUATION. EX. 9/10 9 NODES OUT OF 10 NODES CAN GET LOGIC CONTROL DATA.

| | A | B | C | D | E |
|---|---|---|---|---|---|
| A | START 1(UP) | 1(UP) | 1(UP) | 2(DOWN) | 2(DOWN) |

REPEAT SIMILARLY FROM B,C,D,E TO GET LOGIC CONTROL DATA.

| | A | B | C | D | E |
|---|---|---|---|---|---|
| A | START 1(UP) | 1(UP) | 1(UP) | 2(DOWN) | 2(DOWN) |
| B | 1(UP) | START 1(UP) | 1(UP) | 2(DOWN) | 2(DOWN) |
| C | 1(UP) | 1(UP) | START 1(UP) | 2(DOWN) | 2(DOWN) |
| D | 2(DOWN) | 2(DOWN) | 2(DOWN) | START 1(UP) | 1(UP) |
| E | 2(DOWN) | 2(DOWN) | 2(DOWN) | 1(UP) | START 1(UP) |

# FIG. 6

(a)

PATHWAY DATA

(b) NODE ATTRIBUTE DATA

| ID | INDICATION | ATTRIBUTE | NAME |
|----|------------|-----------|--------|
| 1 | A | GENE | A GENE |
| 2 | B | GENE | B GENE |
| 3 | C | GENE | C GENE |
| 4 | D | GENE | D GENE |
| 5 | E | GENE | E GENE |

(c) EDGE CONTROL DATA

| ID | EDGE NAME | FROM ID | TO ID | CONTROL |
|----|-----------|---------|-------|-----------|
| 1 | $\alpha$ | 1 | 2 | ACTIVATE |
| 2 | $\beta$ | 1 | 3 | ACTIVATE |
| 3 | $\gamma$ | 3 | 4 | INACTIVATE |
| 4 | $\delta$ | 4 | 5 | ACTIVATE |

(d) GRAPHIC/COORDINATES DATA
CONFIGURATION DATA

| CLASSIFICATION | TYPE | CONFIGURATION |
|----------------|---------|---------------|
| NODE | GENE | HEXAGON |
| | CHEMICAL | SQUARE |
| | PROTEIN | ELLIPSE |
| | COMPLEX | DIAMOND |
| EDGE | ACTIVE | ⟶ |
| | INACTIVE | ⊥ |

NODE COORDINATES DATA

| ID | COORDINATES |
|----|-------------|
| 1 | (X1,Y1) |
| 2 | (X2,Y2) |
| 3 | (X3,Y3) |
| 4 | (X4,Y4) |
| 5 | (X5,Y5) |

EP 1 742 161 A1

EP 1 742 161 A1

## FIG. 7

(e)

**PATH-FINDING DATA (FORWARD DIRECTION)**

| | ROUTE DATA(START→END) | | | |
|---|---|---|---|---|
| ROUTE ID | 1 | 2 | 3 | 4 |
| 1 | 1(A) | 2(B) | | |
| 2 | 1(A) | 3(C) | 4(D) | 5(E) |

(f)

**PATHWAY SIMULATION DATA(TWO-TERM RELATIONSHIP)**

| | ROUTE DATA(START→END) +CONTROL DATA FROM A | | | | | | |
|---|---|---|---|---|---|---|---|
| ROUTE ID | 1 | | 2 | | 3 | | 4 |
| 1 | 1(A) UP | ACTIVATE $\alpha$ | 2(B) UP | | | | |
| 2 | 1(A) UP | ACTIVATE $\beta$ | 3(C)UP | INACTIVATE $\gamma$ | 4(D) DOWN | ACTIVATE $\delta$ | 5(E) DOWN |

THE NODE WHICH RECEIVES BOTH UP- AND DOWN-CONTROL IS IN A STATUS OF NO-CHANGE.
UP(1), DOWN(2), NO-CHANGE(0)

ABSENCE OF LOGIC CONTROL DATA GIVES A PENALTY ON THE PATHWAY AND IS TAKEN INTO ACCOUNT ON THE EVALUATION. EX. 9/10 9 NODES OUT OF 10 NODES CAN GET LOGIC CONTROL DATA.

| | A | B | C | D | E |
|---|---|---|---|---|---|
| A | START 1(UP) | 1(UP) | 1(UP) | 2(DOWN) | 2(DOWN) |

REPEAT SIMILARLY FROM B,C,D,E TO GET LOGIC CONTROL DATA.

| | A | B | C | D | E |
|---|---|---|---|---|---|
| A | START 1(UP) | 1(UP) | 1(UP) | 2(DOWN) | 2(DOWN) |
| B | 1(UP) | START 1(UP) | 1(UP) | 2(DOWN) | 2(DOWN) |
| C | 1(UP) | 1(UP) | START 1(UP) | 2(DOWN) | 2(DOWN) |
| D | 2(DOWN) | 2(DOWN) | 2(DOWN) | START 1(UP) | 1(UP) |
| E | 2(DOWN) | 2(DOWN) | 2(DOWN) | 1(UP) | START 1(UP) |

# FIG. 8

**(a)**

### GENE(PROTEIN) EXPRESSION DATA

| SPOT CO-ORDINATES | EXPRESSION RATIO (RATIO VALUE) | | | |
|---|---|---|---|---|
| | TIME-POINT 1 | TIME-POINT 2 | TIME-POINT 3 | TIME-POINT 4 |
| (1,1) | 2.5 | 0.9 | 0.2 | 3.5 |
| (1,2) | 0.2 | 4.0 | 0.2 | 0.2 |
| (1,3) | 0.1 | 5.0 | 0.3 | 4.0 |
| (1,4) | 3.0 | 0.6 | 2.5 | 0.3 |
| (1,5) | 0.9 | 3.0 | 6.0 | 5.0 |

DETERMINATION OF VARIABLE CONDITIONS (EXAMPLE)
UP RATIO ≥ 2.0 → BLACK
DOWN RATIO ≤ 0.5 → GRAY
NO-CHANGE OTHER THAN ABOVE → WHITE

| SPOT CO-ORDINATES | EXPRESSION RATIO (RATIO VALUE) | | | |
|---|---|---|---|---|
| | TIME-POINT 1 | TIME-POINT 2 | TIME-POINT 3 | TIME-POINT 4 |
| (1,1) | 2.5 | 0.9 | 0.2 | 3.5 |
| (1,2) | 0.2 | 4.0 | 0.2 | 0.2 |
| (1,3) | 0.1 | 5.0 | 0.3 | 4.0 |
| (1,4) | 3.0 | 0.6 | 2.5 | 0.3 |
| (1,5) | 0.9 | 3.0 | 6.0 | 5.0 |

**(b)**

### MICROARRAY-DESIGNING DATA

| SPOT CO-ORDINATES | GENE NAME |
|---|---|
| (1,1) | GENE A |
| (1,2) | GENE B |
| (1,3) | GENE C |
| (1,4) | GENE D |
| (1,5) | GENE E |

APPLYING TO EXPERIMENTAL DATA

**(c)**

### PATHWAY NODE ATTRIBUTE DATA

| ID | INDICATION | ATTRIBUTE | NAME |
|---|---|---|---|
| 1 | A | GENE | A GENE |
| 2 | B | GENE | B GENE |
| 3 | C | GENE | C GENE |
| 4 | D | GENE | D GENE |
| 5 | E | GENE | E GENE |

MAPPING TO THE PATHWAY

**(d)**

### PATHWAY NODE ATTRIBUTE DATA + EXPERIMENTAL VALUES (GENE EXPRESSION DATA)

| ID | INDICATION | ATTRIBUTE | NAME | GENE EXPRESSION DATA | | | |
|---|---|---|---|---|---|---|---|
| | | | | TIME-POINT 1 | TIME-POINT 2 | TIME-POINT 3 | TIME-POINT 4 |
| 1 | A | GENE | A GENE | 2.5 | 0.9 | 0.2 | 3.5 | ← FROM A |
| 2 | B | GENE | B GENE | 0.2 | 4.0 | 0.2 | 0.2 |
| 3 | C | GENE | C GENE | 0.1 | 5.0 | 0.3 | 4.0 |
| 4 | D | GENE | D GENE | 3.0 | 0.6 | 2.5 | 0.3 |
| 5 | E | GENE | E GENE | 0.9 | 3.0 | 6.0 | 5.0 |

**(e)**

PATHWAY SIMULATION DATA
(TWO-TERM RELATIONSHIP)
LOGIC DATA IN CASE FROM A

| CONTROL CASE 1 | A | B | C | D | E |
|---|---|---|---|---|---|
| A | START 1 DOWN | | | 2 DOWN | 2 DOWN |

| CONTROL CASE 2 | A | B | C | D | E |
|---|---|---|---|---|---|
| A | START 1 DOWN | 2 DOWN | 2 DOWN | | |

**(f)**

SEARCH FOR EXPRESSION DATA FITTING IN WITH LOGIC DATA
SEARCHING CYCLICALLY FROM A,
NEAREST TIME-POINT FIRST

| A | B | C | D | E | SCORE | CONTROL CASE |
|---|---|---|---|---|---|---|
| | | | ④0.3DOWN | NONE | 2.25 | 1 |
| ②0.9N | NONE | NONE | NONE | NONE | 4.00 | NONE |
| ③0.2DOWN | ③0.2DOWN | ③0.3DOWN | | | 0.00 | 2 |
| | | | ④0.3DOWN | NONE | 3.50 | 1 |

SCORE(EXAMPLE) NODE-SCORE=
|TIME-DISTANCE(PRESENT−DETECTED-TIME)|÷NUMBER-OF-POINTS
THE CASE OF TIME-POINT 1 FOR EXAMPLE
NODE-B-SCORE = (②−①)/4=0.25
NODE-C-SCORE = (②−①)/4=0.25
NODE-D-SCORE = (④−①)/4=0.75
NODE-E-SCORE = NONE=1.0    SUM B~E=2.25
ADD 2 IN CASE OF CYCLIC PENALTY

# FIG. 9

EP 1 742 161 A1

**(a)**

## GENE(PROTEIN) EXPRESSION DATA

| SPOT CO-ORDINATES | EXPRESSION RATIO (RATIO VALUE) | | | |
|---|---|---|---|---|
| | TIME-POINT 1 | TIME-POINT 2 | TIME-POINT 3 | TIME-POINT 4 |
| (1,1) | 2.5 | 0.9 | 0.2 | 3.5 |
| (1,2) | 0.2 | 4.0 | 0.2 | 0.2 |
| (1,3) | 0.1 | 5.0 | 0.3 | 4.0 |
| (1,4) | 3.0 | 0.6 | 2.5 | 0.3 |
| (1,5) | 0.9 | 3.0 | 6.0 | 5.0 |

DETERMINATION OF VARIABLE CONDITIONS (EXAMPLE)
UP RATIO ≧ 2.0 → BLACK
DOWN RATIO ≦ 0.5 → GRAY
NO-CHANGE OTHER THAN ABOVE → WHITE

| SPOT CO-ORDINATES | EXPRESSION RATIO (RATIO VALUE) | | | |
|---|---|---|---|---|
| | TIME-POINT 1 | TIME-POINT 2 | TIME-POINT 3 | TIME-POINT 4 |
| (1,1) | 2.5 | 0.9 | 0.2 | 3.5 |
| (1,2) | 0.2 | 4.0 | 0.2 | 0.2 |
| (1,3) | 0.1 | 5.0 | 0.3 | 4.0 |
| (1,4) | 3.0 | 0.6 | 2.5 | 0.3 |
| (1,5) | 0.9 | 4.0 | 6.0 | 5.0 |

**(b)**

## MICROARRAY-DESIGNING DATA

| SPOT CO-ORDINATES | GENE NAME |
|---|---|
| (1,1) | GENE A |
| (1,2) | GENE B |
| (1,3) | GENE C |
| (1,4) | GENE D |
| (1,5) | GENE E |

APPLYING TO EXPERIMENTAL DATA

**(c)**

## PATHWAY NODE ATTRIBUTE DATA

| ID | INDICATION | ATTRIBUTE | NAME |
|---|---|---|---|
| 1 | A | GENE | A GENE |
| 2 | B | GENE | B GENE |
| 3 | C | GENE | C GENE |
| 4 | D | GENE | D GENE |
| 5 | E | GENE | E GENE |

MAPPING TO THE PATHWAY

# FIG. 10

**(d)**

**PATHWAY NODE ATTRIBUTE DATA + EXPERIMENTAL VALUES (GENE EXPRESSION DATA)**

| ID | INDICATION | ATTRIBUTE | NAME | GENE EXPRESSION DATA | | | |
|---|---|---|---|---|---|---|---|
| | | | | TIME-POINT 1 | TIME-POINT 2 | TIME-POINT 3 | TIME-POINT 4 |
| 1 | A | GENE | A GENE | | 0.9 | 0.2 | |
| 2 | B | GENE | B GENE | 0.2 | | 0.2 | 0.2 |
| 3 | C | GENE | C GENE | 0.1 | | 0.3 | |
| 4 | D | GENE | D GENE | | 0.6 | | 0.3 |
| 5 | E | GENE | E GENE | 0.9 | | | |

←FROM A

**(e)**

**PATHWAY SIMULATION DATA (TWO-TERM RELATIONSHIP) LOGIC DATA IN CASE FROM A**

| CONTROL CASE 1 | A | B | C | D | E |
|---|---|---|---|---|---|
| A | START 1 (UP) | 1 (UP) | 1 (UP) | 2 (DOWN) | 2 (DOWN) |

| CONTROL CASE 2 | A | B | C | D | E |
|---|---|---|---|---|---|
| A | START 2 (DOWN) | 2 (DOWN) | 2 (DOWN) | 1 (UP) | 1 (UP) |

**(f)**

**SEARCH FOR EXPRESSION DATA FITTING IN WITH LOGIC DATA SEARCHING CYCLICALLY FROM A, NEAREST TIME-POINT FIRST**

| A | B | C | D | E | SCORE | CONTROL CASE |
|---|---|---|---|---|---|---|
| ①2.5UP | ②4.0UP | ②5.0UP | ④0.3DOWN | NONE | 2.25 | 1 |
| ②0.9N | NONE | NONE | NONE | NONE | 4.00 | NONE |
| ③0.2DOWN | ③0.2DOWN | ③0.3DOWN | ①2.5UP | ③5.0UP | 0.00 | 2 |
| ④3.5UP | ④4.0UP | ④4.0UP | ④0.3DOWN | NONE | 3.50 | 1 |

SCORE(EXAMPLE)  NODE-SCORE=
   |TIME-DISTANCE(PRESENT−DETECTED-TIME)|÷NUMBER-OF-POINTS
THE CASE OF TIME-POINT 1 FOR EXAMPLE
NODE-B-SCORE = (②−①)/4=0.25
NODE-C-SCORE = (②−①)/4=0.25
NODE-D-SCORE = (④−①)/4=0.75
NODE-E-SCORE = NONE=1.0     SUM B~E=2.25
ADD 2 IN CASE OF CYCLIC PENALTY

# FIG. 11

**PATHWAY NODE ATTRIBUTE DATA + EXPERIMENTAL VALUES (GENE EXPRESSION DATA)**

| ID | INDI-CATION | ATTRIBUTE | NAME | GENE EXPRESSION DATA | | | |
|----|-------------|-----------|------|---------------|---------------|---------------|---------------|
| | | | | TIME-POINT 1 | TIME-POINT 2 | TIME-POINT 3 | TIME-POINT 4 |
| 1 | A | GENE | A GENE | 2.5 | 0.9 | 0.2 | 3.5 |
| 2 | B | GENE | B GENE | 0.2 | 4.0 | 0.2 | 0.2 |
| 3 | C | GENE | C GENE | 0.1 | 5.0 | 0.3 | 4.0 |
| 4 | D | GENE | D GENE | 3.0 | 0.6 | 2.5 | 0.3 |
| 5 | E | GENE | E GENE | 0.9 | 3.0 | 6.0 | 5.0 |

**PATHWAY GRAPH DATA (ORIGINAL COORDINATES/ CONFIGURATION)**

**SEARCH FOR EXPRESSION DATA FITTING IN WITH LOGIC DATA**
**SEARCHING CYCLICALLY FROM A, NEAREST TIME-POINT FIRST**

| A | B | C | D | E | SCORE | CONTROL CASE |
|---|---|---|---|---|-------|--------------|
| ①2.5 UP | ④4.0 UP | ②2.5 UP | ④0.3 DOWN | NONE | 2.25 | 1 |
| ②0.9 N | NONE | NONE | NONE | NONE | 4.00 | NONE |
| ③0.2 DOWN | ③0.2 DOWN | ③0.3 DOWN | ③2.5 UP | ③6.0 UP | 0.00 | 2 |
| ④3.5 UP | ②4.0 UP | ①4.0 UP | ④0.3 DOWN | NONE | 3.50 | 1 |
| GROSS EVALUATION TOTAL SCORE | | | | | 9.75 | |

**GRAPH DATA (EXAMPLE TIME-POINT 1)**

| GENE/ TIME-POINT | CURRENT(1) | TIME-POINT 1 | TIME-POINT 2 | TIME-POINT 3 | TIME-POINT 4 |
|------------------|------------|--------------|--------------|--------------|--------------|
| A | 2.5UP HEXAGON | 2.5UP HEXAGON | 0.9NO-CHANGE EMPHASIZED CONFIGURATION | 0.2DOWN HEXAGON | 3.5UP HEXAGON |
| B | 0.2DOWN HEXAGON | 0.2DOWN HEXAGON | 4.0UP HEXAGON | 0.2DOWN HEXAGON | 0.2DOWN HEXAGON |
| C | 0.1DOWN HEXAGON | 0.1DOWN HEXAGON | 5.0UP HEXAGON | 0.3DOWN HEXAGON | 4.0UP HEXAGON |
| D | 3.0UP HEXAGON | 3.0UP HEXAGON | 0.6NO-CHANGE EMPHASIZED CONFIGURATION | 2.5UP HEXAGON | 0.3DOWN HEXAGON |
| E | 0.9NO-CHANGE EMPHASIZED CONFIGURATION | 0.9NO-CHANGE EMPHASIZED CONFIGURATION | 3.0UP HEXAGON | 6.0UP HEXAGON | 5.0UP HEXAGON |

IMAGING

ANIMATING CYCLIC TIME1 → TIME2 → TIME3 → TIME4 → TIME1

# FIG. 12

**PATHWAY NODE ATTRIBUTE DATA + EXPERIMENTAL VALUES (GENE EXPRESSION DATA)**

| ID | INDI-CATION | ATTRIBUTE | NAME | GENE EXPRESSION DATA | | | |
|---|---|---|---|---|---|---|---|
| | | | | TIME-POINT 1 | TIME-POINT 2 | TIME-POINT 3 | TIME-POINT 4 |
| 1 | A | GENE | A GENE | 2.5 | 0.9 | 0.2 | 3.5 |
| 2 | B | GENE | B GENE | 0.2 | 4.0 | 0.2 | 0.2 |
| 3 | C | GENE | C GENE | 0.1 | 5.0 | 0.3 | 4.0 |
| 4 | D | GENE | D GENE | 3.0 | 0.6 | 2.5 | 0.3 |
| 5 | E | GENE | E GENE | 0.9 | 3.0 | 6.0 | 5.0 |

**PATHWAY GRAPH DATA (ORIGINAL COORDINATES/ CONFIGURATION)**

A —α→ B
β γ δ
C — D — E

**SEARCH FOR EXPRESSION DATA FITTING IN WITH LOGIC DATA**
**SEARCHING CYCLICALLY FROM A, NEAREST TIME-POINT FIRST**

| A | B | C | D | E | SCORE | CONTROL CASE |
|---|---|---|---|---|---|---|
| ①2.5 UP | ②4.0 UP | ②5.0 UP | ④0.3 DOWN | NONE | 2.25 | 1 |
| ②0.9 N | NONE | NONE | NONE | NONE | 4.00 | NONE |
| ③0.2 DOWN | ③0.2 DOWN | ③0.3 DOWN | ③2.5 UP | ③6.0 UP | 0.00 | 2 |
| ④3.5 UP | ②4.0 UP | ④4.0 UP | ④0.3 DOWN | NONE | 3.50 | 1 |
| GROSS EVALUATION TOTAL SCORE | | | | | 9.75 | |

**GRAPH DATA (EXAMPLE TIME-POINT 1)**

| GENE/ TIME-POINT | CURRENT(1) | TIME-POINT 1 | TIME-POINT 2 | TIME-POINT 3 | TIME-POINT 4 |
|---|---|---|---|---|---|
| A | 2.5UP HEXAGON | 2.5UP HEXAGON | 0.9NO-CHANGE EMPHASIZED CONFIGURATION | 0.2DOWN HEXAGON | 3.5UP HEXAGON |
| B | 0.2DOWN HEXAGON | 0.2DOWN HEXAGON | 4.0UP HEXAGON | 0.2DOWN HEXAGON | 0.2DOWN HEXAGON |
| C | 0.1DOWN HEXAGON | 0.1DOWN HEXAGON | 5.0UP HEXAGON | 0.3DOWN HEXAGON | 4.0UP HEXAGON |
| D | 3.0UP HEXAGON | 3.0UP HEXAGON | 0.6NO-CHANGE EMPHASIZED CONFIGURATION | 2.5UP HEXAGON | 0.3DOWN HEXAGON |
| E | 0.9NO-CHANGE EMPHASIZED CONFIGURATION | 0.9NO-CHANGE EMPHASIZED CONFIGURATION | 3.0UP HEXAGON | 6.0UP HEXAGON | 5.0UP HEXAGON |

IMAGING → A → B, C → D → E

ANIMATING
TIME1 → TIME2 → TIME3 → TIME4 CYCLIC

# FIG. 13

| EXPRESSION LEVEL U(UP),D(DOWN), N(NO-CHANGE) | TIME-POINT/SAMPLE | | | |
|---|---|---|---|---|
| GENE/PROTEIN | ① | ② | ③ | ④ |
| A | U | N | D | U |
| B | D | U | D | D |
| C | D | U | D | U |
| D | U | N | U | D |
| E | N | U | U | U |

LIST OF LOG SCORE

TIME-POINT3 SCORE 0.00

TIME-POINT1 SCORE 2.25

TIME-POINT4 SCORE 3.50

TIME-POINT2 SCORE 4.00

GROSS EVALUATION 9.75

## FIG. 14

| | TYPES OF EDGES(RESULT) | CHANGES IN STATUS (LEFT OF THE ARROW IS THE STATUS OF CONTROLLING GENE, RIGHT OF THE ARROW IS THE STATUS OF CONTROLLED GENE) |
|---|---|---|
| 1 | ACTIVATION | ACTIVE STATUS  UP            < − > ACTIVE STATUS  UP,<br>ACTIVE STATUS  DOWN         − > ACTIVE STATUS  DOWN,<br>EXPRESSION LEVEL  UP         − > ACTIVE STATUS  UP,<br>EXPRESSION LEVEL  DOWN     − > ACTIVE STATUS  DOWN |
| 2 | INHIBITION/INACTIVATION | ACTIVE STATUS  UP            < − >ACTIVE STATUS  DOWN,<br>ACTIVE STATUS  DOWN         − >ACTIVE STATUS  UP,<br>EXPRESSION LEVEL  UP         − >ACTIVE STATUS  DOWN,<br>EXPRESSION LEVEL  DOWN     − >ACTIVE STATUS  UP |
| 3 | EXPRESSION | ACTIVE STATUS  UP             < − >EXPRESSION LEVEL  UP,<br>ACTIVE STATUS  DOWN          − >EXPRESSION LEVEL  DOWN,<br>EXPRESSION LEVEL  UP        < − >EXPRESSION LEVEL  UP,<br>EXPRESSION LEVEL  DOWN    < − >EXPRESSION LEVEL  DOWN |
| 4 | REPRESSION | ACTIVE STATUS  UP             < − >EXPRESSION LEVEL  DOWN,<br>ACTIVE STATUS  DOWN          − >EXPRESSION LEVEL  UP,<br>EXPRESSION LEVEL  UP        < − >EXPRESSION LEVEL  DOWN,<br>EXPRESSION LEVEL  DOWN    < − >EXPRESSION LEVEL  UP |
| 5 | COMPLEX | NO-CHANGE |
| 6 | NO-CHANGE/UNKNOWN | NO-CHANGE |

EP 1 742 161 A1

# FIG. 15

| NO | CONNECTION CATEGORY | TYPE OF CONNECTION | STATUS | NOTATION(FORM) |
|---|---|---|---|---|
| | | TYPES OF EDGES, CONTROL SYSTEMS, EDGE FORMS | | |
| 1 | CAUSE | BINDING/ ASSOCIATION | ASSOCIATION OF PROTEIN/DNA OR PROTEIN/PROTEIN | "BI" FOR EDGE NAME  BI ——→  CAUSE CATEGORY OF N0.1-16 FORMS AN EDGE LEADING RESULT CATEGORY OF NO.17-23 |
| 2 | CAUSE | DISSOCIATION | DISSOCIATION OF PROTEIN/DNA OR PROTEIN/PROTEIN | "DS" FOR EDGE NAME |
| 3 | CAUSE | PHOSPHORILATION | PHOSPHORILATION OF PROTEIN | "+P" FOR EDGE NAME |
| 4 | CAUSE | DEPHOSPHORILATION | DEPHOSPHORILATION OF PROTEIN | "−P" FOR EDGE NAME |
| 5 | CAUSE | UBIQUITINATION | UBIQUITINATION OF PROTEIN | "+U" FOR EDGE NAME |
| 6 | CAUSE | CLEAVAGE | CLEAVAGE OF PROTEIN | "CL" FOR EDGE NAME |
| 7 | CAUSE | ACETYLATION | ACETYLATION OF PROTEIN | "+A" FOR EDGE NAME |
| 8 | CAUSE | DEACETYLATION | DEACETYLATION OF PROTEIN | "−A" FOR EDGE NAME |
| 9 | CAUSE | GLYCOSYLATION | GLYCOSYLATION OF PROTEIN | "+G" FOR EDGE NAME |
| 10 | CAUSE | DEGLYCOSYLATION | DEGLYCOSYLATION OF PROTEIN | "−G" FOR EDGE NAME |
| 11 | CAUSE | ALKYLATION | ALKYLATION OF DNA | "+R" FOR EDGE NAME |
| 12 | CAUSE | DEALKYLATION | DEALKYLATION OF DNA | "−R" FOR EDGE NAME |
| 13 | CAUSE | METHYLATION | METHYLATION OF DNA | "+M" FOR EDGE NAME |
| 14 | CAUSE | DEMETHYLATION | DEMETHYLATION OF DNA | "−M" FOR EDGE NAME |
| 15 | CAUSE | MOLTRANSPORT | MOLTRANSPORT | "TR" FOR EDGE NAME |
| 16 | CAUSE | REGULATION | CASE OF REGULATION OR UNKNOWN ORIGIN | "RE" FOR EDGE NAME |
| 17 | RESULT | ACTIVATION | ACTIVATION OR FUNCTION ACQUIRING OF PROTEIN | ——→ |
| 18 | RESULT | INHIBITION/ INACTIVATION | INACTIVATION OR MALFUNCTION OF PROTEIN | ——⊣ |
| 19 | RESULT | EXPRESSION | INCREASE OF GENE EXPRESSION | ·····►|
| 20 | RESULT | REPRESSION | DECREASE OF GENE EXPRESSION | ·····⊣ |
| 21 | RESULT | NO DESCRIPTION, NO CHANGE, ETC. | NO DESCRIPTION, NO CHANGE, ETC. | ——▷ |
| 22 | RESULT | COMPLEX | OMPLEX(INCLUDING HETERODIMER AND HOMODIMER) | ——● |
| 23 | RESULT | DEGRADATION/ PROTEOLYSIS | DEGRADATION OF PROTEIN | —‖→ |

FIG. 16

FIG. 17

EP 1 742 161 A1

FIG. 18

A

| TEST7 |
| TEST6 |
| TEST5 |
| TEST4 |

B

| TEST8 |
| TEST7 |
| TEST6 |
| TEST5 |
| TEST4 |

C

| TEST5 |
| TEST4 |
| TEST1 |

D

| TEST1 |

EP 1 742 161 A1

**European Patent Office**

## DECLARATION

which under Rule 45 of the European Patent Convention shall be considered, for the purposes of subsequent proceedings, as the European search report

**Application Number**

EP 05 25 6350

| The Search Division considers that the present application, does not comply with the provisions of the EPC to such an extent that it is not possible to carry out a meaningful search into the state of the art on the basis of all claims | CLASSIFICATION OF THE APPLICATION (IPC) |
|---|---|
| Reason: | INV. G06F19/00 |

The present application contains 25 claims, of which 15 are independent. There is no clear distinction between the independent claims because of overlapping scope. There are so many claims, and they are drafted in such a way that the claims as a whole are not in compliance with the provisions of clarity and conciseness of Article 84 EPC, as it is particularly burdensome for a skilled person to establish the subject-matter for which protection is sought. The non-compliance with the substantive provisions is to such an extent, that a meaningful search of the whole claimed subject-matter could not be carried out (Rule 45 EPC and Guidelines B-VIII, 3).

The applicant's attention is drawn to the fact that a search may be carried out during examination following a declaration of no search under Rule 45 EPC, should the problems which led to the declaration being issued be overcome (see EPC Guideline C-VI, 8.5).

-----

| Place of search | Date | Examiner |
|---|---|---|
| Munich | 17 July 2006 | Barba, M |

EPO FORM 1504 (P04C37)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2002025489 A